(19) Europäisches Patentamt
European Patent Office
Office européen des brevets

(11) Numéro de publication: **0 490 772 B1**

(12) # FASCICULE DE BREVET EUROPEEN

(45) Date de publication de fascicule du brevet: **26.07.95**

(51) Int. Cl.[6]: **C07D 319/18**, C07D 321/10, C07D 307/79, A61K 31/495

(21) Numéro de dépôt: **91403378.2**

(22) Date de dépôt: **13.12.91**

(54) **Piperazines 1,4-disubstiuées, leur procédé de préparation et les compositions pharmaceutiques les renfermant.**

(30) Priorité: **14.12.90 FR 9015631**

(43) Date de publication de la demande:
**17.06.92 Bulletin 92/25**

(45) Mention de la délivrance du brevet:
**26.07.95 Bulletin 95/30**

(84) Etats contractants désignés:
**AT BE CH DE DK ES FR GB GR IT LI LU NL SE**

(56) Documents cités:
**EP-A- 0 241 654**
**EP-A- 0 376 607**
**US-A- 3 466 287**
**US-A- 3 678 059**

**Patent Abstracts of Japan vol. 10 no.280(C-374)(2336), 24 september 1986.**

**Chemical Abstracts vol.95, no. 1, 6 July 1981, page 685, col. 1, Columbus Ohio, US; VE Golubev et al.**

**Archives Internationales de Pharmacodynamie et de Therapie, vol. 157, no.1, 1965, pages 67-89, Brugge, B; WB Mckeon.**

(73) Titulaire: **ADIR ET COMPAGNIE**
**1 rue Carle Hébert**
**F-92415 Courbevoie Cédex (FR)**

(72) Inventeur: **Peglion, Jean-Louis**
**5 Allée des Bégonias**
**F-78110 Le Vesinet (FR)**
Inventeur: **Millan, Mark**
**179 Bld. Malesherbes**
**F-75017 Paris (FR)**
Inventeur: **Rivet, Jean-Michel**
**160 Rue de la Source**
**F-92000 Nanterre (FR)**

(74) Mandataire: **Reverbori, Marcelle**
**ADIR**
**1, rue Carle Hébert**
**F-92415 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 490 772 B1

## Description

La présente invention a pour objet de nouvelles pipérazines 1,4-disubstituées, leur procédé de préparation et les compositions pharmaceutiques les renfermant.

Elle concerne particulièrement les pipérazines 1,4-disubstituées de formule générale I :

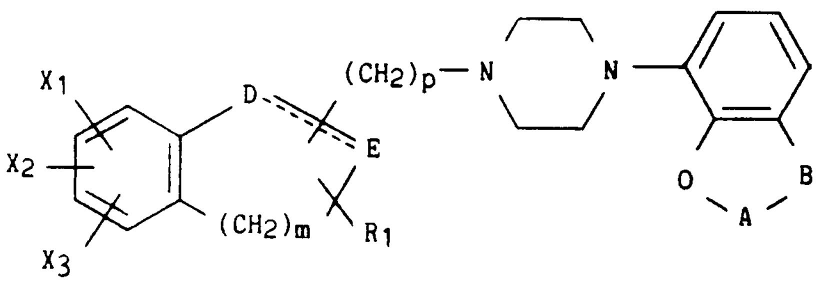

(I)

dans laquelle :

- $X_1$, $X_2$ et $X_3$ :
  - identiques ou différents, représentent chacun : un atome d'hydrogène ou d'halogène, un radical alkyle en chaine droite ou ramifiée contenant de 1 à 5 atomes de carbone, un radical hydroxy, un radical alcoxy ou alkylthio contenant chacun de 1 à 5 atomes de carbone en chaine droite ou ramifiée, un radical trifluorométhyle, un radical nitro, un radical amino, ou un radical acétamido, ou
  - deux d'entre eux pris en position adjacente forment ensemble un radical méthylènedioxy ou un radical éthylènedioxy ;
- $R_1$ représente un atome d'hydrogène ou un radical alkyle en chaine droite ou ramifiée contenant de 1 à 5 atomes de carbone ;
- - D$=\!=$E - représente : $-(CH_2)_n-(CH_2)-$ ou $-CH=CH-$
- m et n représentent chacun les valeurs zéro, un, deux ou trois à condition que m + n soit $\geqq 1$.
- p représente zéro ou un nombre entier de 1 à 6, et
- -A-B- représente un des radicaux de formule :
  $-(CH_2)_2-O-$ ; $-(CH_2)_3-O-$ ; $-CH=CH-$ ; $-CH_2-CH_2-$ et

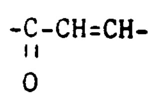

.

Certains dérivés de formule générale I renferment un atome de carbone asymétrique et de ce fait peuvent être dédoublés en isomères optiques lesquels sont également inclus dans la présente invention.

L'état antérieur de la technique dans ce domaine est illustré notamment par les demandes de brevet européen publiées sous les N° 138.280 ; 185.429 ; 189.612 ; 307.061 et 376.607.

Aucune de ces demandes ne décrit ni suggère les dérivés objet de la présente invention, lesquels dérivés ont une activité pharmacologique du type antagoniste 5 $HT_1$ A, ce qui n'est pas le cas des dérivés de l'état antérieur de la technique ci-dessus cité.

La présente invention a également pour objet le procédé de préparation des dérivés de formule générale I, caractérisé en ce que l'on condense :

- une pipérazine N-monosubstituée de formule générale II :

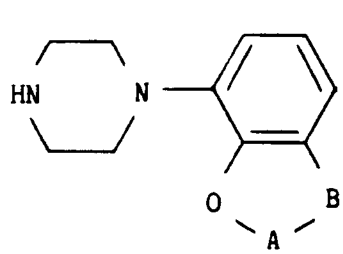

(II)

dans laquelle le groupe -A-B- a la signification précédemment définie, avec :

- un dérivé de formule générale III :

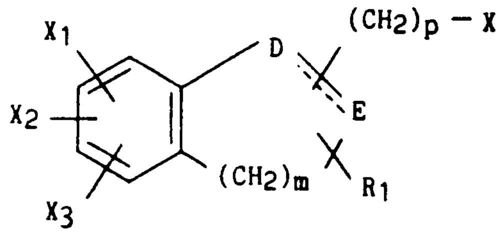

(III)

dans laquelle :

- $X_1$, $X_2$, $X_3$, $R_1$, - D=E-, m et p ont les significations précédemment définies et
- X représente un atome d'halogène, ou un radical mésyloxy ou tosyloxy.

La condensation s'effectue de façon particulièrement adéquate en opérant dans un solvant approprié tel que , par exemple, la méthyléthylcétone, la méthylisobutylcétone, le toluène, ou le diméthylformamide en présence d'un accepteur de l'acide formé au cours de la réaction, à une température de 20 à 150 °C. Comme accepteur, on peut employer par exemple un carbonate de métaux alcalins comme le carbonate de sodium ou une amine tertiaire comme la triéthylamine.

De plus, les dérivés de formule générale I dans laquelle p prend les significations autre que zéro, c'est à dire les dérivés répondant plus précisément à la formule générale I' :

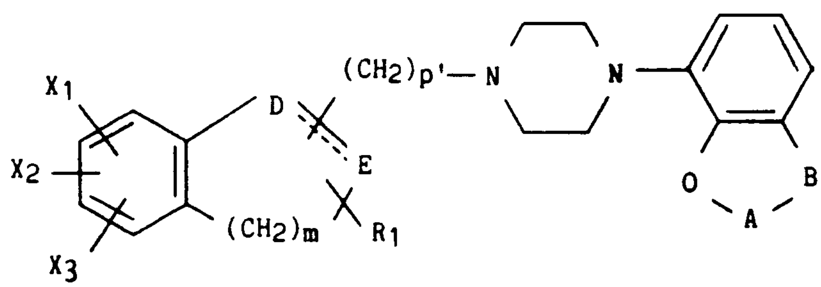

(I')

dans laquelle :

- $X_1$, $X_2$, $X_3$, $R_1$, - D=E - , m et -A-B- ont les significations précédemment définies et
- p' représente un nombre entier de 1 à 6, ont également été préparés selon une variante du procédé précédent caractérisée en ce que :

l'on condense :

- la pipérazine N-monosubstituée de formule générale II précédemment définie, avec
- un dérivé de formule générale IV :

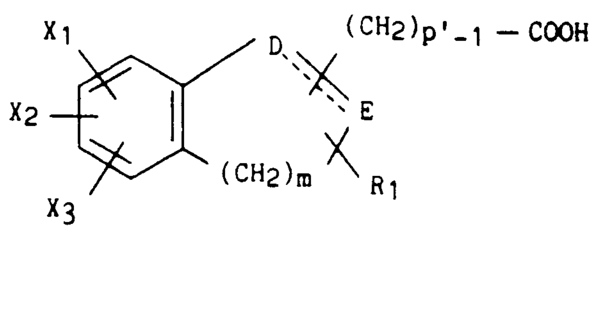

(IV)

dans laquelle :

- $X_1$, $X_2$, $X_3$, $R_1$, - D=E- , m et p' ont les significations précédemment définies ; et

- l'on réduit l'amide ainsi obtenue de formule générale V :

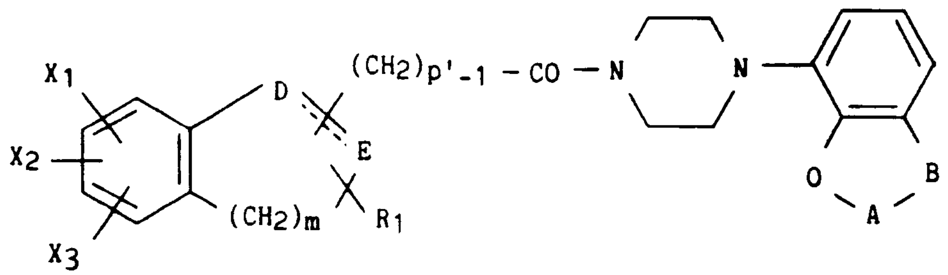

(V)

dans laquelle :

- $X_1$, $X_2$, $X_3$, $R_1$, - D=E- , m, p' et -A-B- ont les significations précédemment définies .

La condensation des dérivés II et IV s'effectue de façon particulièrement adéquate en opérant dans un solvant approprié comme par exemple le chlorure de méthylène, en présence de carbonyldiimidazole.

La réduction de l'amide V s'effectue avantageusement au moyen d'un hydrure double de lithium-aluminium dans un solvant adéquat comme par exemple l'éther ou le tétrahydrofurane.

Ce dernier procédé de préparation des dérivés I' est également inclus dans la présente invention.

De plus, les amides de formule générale V sont des produits intermédiaires nouveaux qui font, à ce titre, partie de la présente invention.

Les matières premières de formules II, III et IV sont soit des produits connus, soit des produits préparés à partir de composés connus selon des procédés connus, comme précisé dans les exemples ci-après.

Les dérivés de formule générale I donnent des sels avec les acides physiologiquement tolérables. Ces sels sont également inclus dans la présente invention.

Les dérivés de la présente invention possèdent des propriétés pharmacologiques et thérapeutiques intéressantes. En effet, les essais pharmacologiques ont démontré que les composés de l'invention se comportent, in vitro et in vivo, comme des ligands très puissants et très sélectifs des récepteurs de la sérotonine $5HT_{1A}$ avec une activité antagoniste de ce neurotransmetteur au niveau du système nerveux central, démontrée par l'étude pharmacologique ci-après exemplifiée.

Cette activité permet l'utilisation des dérivés de la présente invention dans le traitement des maladies du système nerveux central, notamment de l'anxiété, la douleur, la dépression, la psychose, la schizophrénie, la migraine, les troubles de la cognition, le stress et l'anorexie et des maladies neuroendocriniennes comme le diabète.

La présente invention a également pour objet les compositions pharmaceutiques contenant comme principe actif un dérivé de formule générale I ou un de ses sels physiologiquement tolérables, mélangé ou associé à un excipient pharmaceutique approprié, comme par exemple, le glucose, le lactose, le talc, l'éthylcellulose, le stéarate de magnésium ou le beurre de cacao.

Les compositions pharmaceutiques ainsi obtenues se présentent généralement sous forme dosée et peuvent contenir de 0,1 à 100 mg de principe actif. Elles peuvent revêtir, par exemple, la forme de comprimés, dragées, gélules, suppositoires, solutions injectables ou buvables et être selon les cas, administrées par voie orale, rectale ou parentérale à la dose de 0,1 à 100 mg de principe actif 1 à 3 fois par jour.

Les exemples suivants illustrent la présente invention, les points de fusion étant déterminés à la platine chauffante de Kofler (K) éventuellement sous microscope (M.K).

**Exemple 1 :**

4-(Benzodioxan-5-yl)-1-[(benzocyclobutan-1-yl)méthyl]-pipérazine (R,S) :

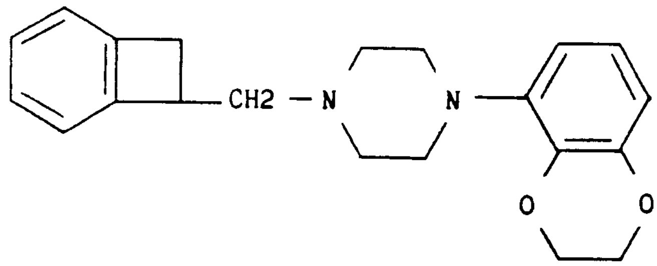

On mélange 4 g (16,4.$10^{-3}$ mole) d'iodure de (benzocyclobutan-1-yl)méthyle, 3,61 g (16,4.$10^{-3}$ mole) de N-(benzodioxan-5-yl)- piperazine, 6,95 g (65,5.$10^{-3}$ mole) de $Na_2CO_3$ et 100 ml de méthylisobutylcétone ; et chauffe le tout à reflux que l'on maintient pendant 24 heures sous agitation. On concentre le mélange réactionnel à l'évaporateur rotatif, reprend le concentrat par $CH_2Cl_2$. Après lavage à l'eau, on extrait la phase organique avec une solution normale d'acide chlorhydrique. On alcalinise la phase aqueuse puis l'extrait au $CH_2Cl_2$. Après séchage et concentration on obtient 4,4 g d'une huile que l'on cristallise dans l'éther. Le solide obtenu est recristallisé dans 15 ml d'éther isopropylique. On obtient 1,6 g de 4-(benzodioxan-5-yl)-1-[(benzocyclobutan-1-yl)méthyl] pipérazine (R,S), P.F.(M.K) : 91-95 °C, Rendement : 29 %, qui a été chromatographié sur couche mince (solvants : chlorure de méthylène-méthanol 90-10).

- RMN (solvant : $CDCl_3$)
  4H (m) 7,3-7,0 ppm ; 1H (t) 6,8 ppm ; 2H (m) 6,6 ppm ; 4H (m) 4,3 ppm ;
  1H (m) 3,7 ppm ; 1H (dd) 3,4 ppm ; 4H (m) 3,10 ppm ; 2H (m) 2,85 ppm ;
  4H (m) 2,7 ppm ; 1H (dd) 2,65 ppm.

La N-(benzodioxan-5-yl)-pipérazine de départ a été préparée selon la méthode décrite dans J. Med. Chem (1988) 31, 1934, à partir du 1-nitro-2,3-dihydroxybenzène, lui-même décrit dans J.A.C.S (1953), 3277.

De la même façon, ont été préparés les dérivés objets des exemples 2 à 5.

**Exemples 2-5 :**

**2)** La 4-(benzodioxan-5-yl)-1[2-(benzocyclobutan-1-yl)éthyl]pipérazine (R,S) et son dichlorhydrate P.F. (M.K) : 215-226 °c (avec sublimation à partir de 192 °C) (Rendement : 56 %), à partir du bromure de 2-(benzocyclobutan-1-yl)éthyle (lui-même préparé à partir de l'alcool correspondant comme décrit dans la demande de brevet français déposée le 7 novembre 1989 sous le n° 89.14571) et de la N-(benzodioxan-5-yl) pipérazine, en présence de $Na_2CO_3$ par chauffage à reflux dans la méthylisobutylcétone pendant 8 heures.

**3)** La 4-[benzo(1,5)dioxépin-6-yl]-1-[2-(benzocyclobutan-1-yl) méthyl pipérazine (R,S) et son chlorhydrate, P.F. (M.K) : 170-210 °C (avec sublimation), Rendement : 51 %, à partir du bromure de 2-(benzocyclobutan-1-yl)éthyle et de la N-(benzo(1,5)dioxépin-6-yl)pipérazine, elle-même décrite dans J. Med. Chem. (1988) 31, 1934.

**4)** La 4-(benzofuran-7-yl)-1-[2-(benzocyclobutan-1-yl)éthyl] pipérazine (R,S), et son chlorhydrate P.F. (M.K) : 192-195 °C (isopropanol) (Rendement : 47%), à partir du bromure de 2-(benzocyclobutan-1-yl) éthyle et de la N-(benzofuran-7-yl) pipérazine, elle-même preparée, avec un rendement de 49 %, selon la méthode décrite dans J. Med. Chem (1988) 31, 1934 à partir du chlorhydrate de di-(2-chloroéthyl)-amine et du chlorhydrate de 7-aminobenzofurane lui-même obtenu par réduction du 7-nitrobenzofurane. Ce dernier a été préparé à partir du 2-éthoxycarbonyl-7-nitrobenzofurane lui-même obtenu à partir du 2-hydroxy-3-nitro benzaldéhyde lui-même formé par nitration du 2- hydroxybenzaldéhyde.

**5)** La 4-(benzodioxan-5-yl)-1-[2-(3-chlorobenzocyclobutan-1-yl)éthyl] pipérazine (R,S) et son dichlorhydrate P.F.(M.K) : 207-211 °C (cyanure de méthyle) à partir du bromure de 2-(3-chlorobenzocyclobutan-1-yl) éthyle (huile $Eb/_{0,1}$ mmHg : 80 °C) et de la N-(benzodioxan-5-yl) pipérazine (Rendement : 54 %). Le bromure de 2-(3-chlorobenzocyclobutan-1-yl)éthyle a été préparé à partir de l'acide (3-chlorobenzocyclobutan-1-yl) carboxylique, qui, traité par $LiAlH_4$ puis le chlorure de tosyle, donne le tosylate de (3-chlorobenzocyclobutan-1-yl)méthyle P.F (K) : 60-62 °C (Rendement : 84 %), lequel traité par le cyanure de sodium dans le diméthylsulfoxyde, puis par l'hydroxyde de potassium dans une solution aqueuse

d'éthanol et enfin réduit en acide (3-chlorobenzocyclobutan-1-yl) méthyl carboxylique, P.F (K) : 94-96 °C, lequel est alors traité par $LiAlH_4$ puis $PBr_3$ dans le benzène pour donner le bromure attendu, avec un rendement de 41 %.

**Exemple 6 :**

4-[Benzo(1,5)dioxépin-6-yl]-1-[(benzocyclobutan-1-yl)méthyl] pipérazine (R,S) :

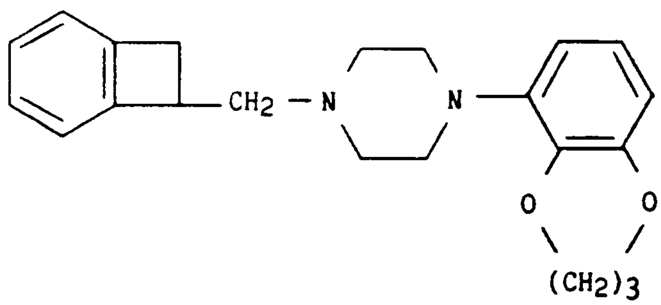

On mélange 4 g ($13,8.10^{-3}$ mole) de tosylate de (benzocyclobutan-1-yl)méthyle, 3,3 g ($13,8.10^{-3}$ mole) de N-benzo(1,5)dioxépin-6-yl)pipérazine, 3,9 ml ($27,6.10^{-3}$ mole) de triéthylamine et 50 ml de toluène ; et chauffe le tout à reflux que l'on maintient pendant 24 heures. On concentre le mélange réactionnel à l'évaporateur rotatif et reprend le concentrat par $H_2O$ et $CH_2Cl_2$. On extrait la phase organique avec une solution normale d'acide chlorhydrique. On alcalinise la phase aqueuse puis l'extrait au $CH_2Cl_2$. Après séchage, on obtient 2,1 g d'un solide que l'on dissout dans 20 ml d'éthanol. A cette solution éthanolique on ajoute 1,7 ml d'éther chlorhydrique 3,5 N et abandonne le tout au réfrigérateur pendant 48 heures. Le précipité formé est filtré, séché. On recueille 2 g de chlorhydrate de 4-[benzo(1,5)dioxépine-6-yl]-1-[-(benzocyclobutan-1-yl)méthyl] pipérazine (R,S), P.F. (M.K) : 248-252 °C (avec sublimation à partir de 190 °C), Rendement : 37 %, qui a été chromatographié sur couche mince (solvants : chlorure de méthylène-méthanol, 95-5).

De la même façon ont été préparés les dérivés objets des exemples 7 à 10.

**Exemple 7-10 :**

**7)** La 4-(benzodioxan-5-yl)-1-(indan-2-yl)pipérazine, P.F. (M.K.) : 168-171 °C, à partir du tosylate d'indan-2-yle [cf. Bull. Soc. Chem. (1962) p 51] et de N-(benzodioxan-5-yl)pipérazine. (Rendement : 11 %).

**8)** La 4-(benzodioxan-5-yl)-1-[4-(benzocyclobutan-1-yl)butyl] pipérazine (R,S) et son fumarate P.F (M.K) : 180-183 °C (éthanol), à partir du mésylate de 4-(benzocyclobutan-1-yl)butyle (huile) et du dichlorhydrate de N-(benzodioxan-5-yl)pipérazine. (Rendement : 44 %).

Le mésylate de 4-(benzocyclobutan-1-yl) butyle a lui-même été préparé, avec un rendement de 96 %, en traitant le 4-(benzocyclobutan-1-yl)butanol (huile) par $CH_3SO_2Cl$ en présence de triéthylamine dans le chlorure de méthylène.

**9)** La 4-[benzo(1,5)dioxépin-6-yl]-1-(indan-2-yl)pipérazine, P.F (M.K) : 138-140 °C, à partir du tosylate d'indan-2 yle et de N-[benzo(1,5)dioxépin-6-yl] pipérazine [cf. J. Med. Chem. (1988) p 1935] (Rendement : 20 %).

**10)** La 4-(coumarin-8-yl)-1-(indan-2-yl)pipérazine, P.F (M.K) : 162-163 °C (acétonitrile) à partir du tosylate d'indan-2 yle et de N-(coumarin-8-yl) pipérazine P.F (K)>260 °C (sublimation). Rendement : 28 %.

La N-(coumarin-8-yl) pipérazine a été préparée en faisant réagir la 8-amino coumarine avec un excès de chlorhydrate de bis (2-chloroéthyl) amine en présence de carbonate de potassium puis d'iodure de potassium, en opérant à reflux dans le chlorobenzène.

La 8-aminocoumarine a été obtenue à partir du dérivé nitré correspondant selon Archiv. der Parmazie, (1963), <u>296</u> (6), 365-369 ;
lequel dérivé nitré a lui-même été préparé à partir de l'o-hydroxybenzaldéhyde, selon Fort. Hase Papers (1975), <u>6</u> (2), 109-118.

**Exemple 11 :**

4-(Benzodioxan-5-yl)-1-[(3-chlorobenzocyclobutan-1-yl)méthyl] pipérazine (R,S)

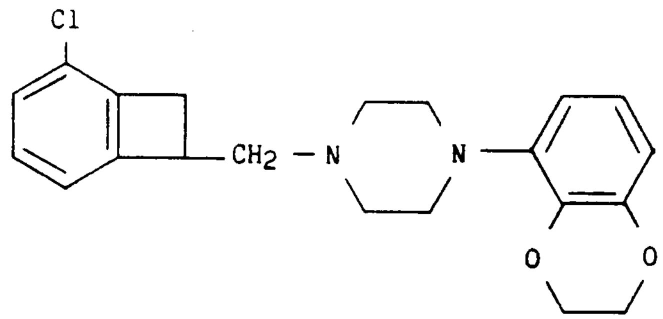

a) première étape :

A 0,1 mole d'acide (3-chlorobenzocyclobutan-1-yl) carboxylique dans 200 ml de chlorure de méthylène on ajoute en une fois, sous atmosphère d'azote, 0,1 mole de N,N-carbonyldiimidazole, et on laisse en contact pendant 2 heures. On ajoute ensuite, en un goutte à goutte rapide, 0,1 mole de N-(benzodioxan-5-yl)pipérazine en solution dans 50 ml de chlorure de méthylène. On laisse en contact sous agitation durant une nuit. Puis on évapore, reprend le résidu à l'éther, extrait la phase organique avec une solution normale d'acide chlorhydrique, puis alcalinise les phases aqueuses à froid.

Après évaporation et chromatographie de l'huile résiduelle (solvants : chlorure de méthylène-acétate d'éthyle, 90-10), on obtient la 4-(benzodioxan-5-yl)-1-[(3-chlorobenzocyclobutan-1-yl)carbonyl] pipérazine (R,S), P.F. (K) : 182-184 °C, Rendement : 40 %.

- RMN (solvant : $CDCl_3$) :
  3H, 7,25 et 7,05 ppm (m) ; 1H, 6,8 ppm (t) ; 2H 6,7 à 6,5 ppm (d) ;
  1H, 4,5 ppm (m) ; 4H, 4,25 à 4 ppm (m) ; 4H, 3,85 ppm ;
  2H, 3,65 et 3,45 ppm (dd) ; 4H, 3,05 ppm (t + t).

L'acide (3-chlorobenzocyclobutan-1-yl)carboxylique de départ a été préparé, comme décrit dans la demande de brevet européen déposée par la demanderesse sous le n° 90.403145.7, à partir du 3-chloro-1-cyanobenzocyclobutane lui-même décrit dans le brevet européen 119.107.

b) deuxième étape

A une suspension de 0,1 mole d'hydrure de lithium aluminium dans 50 ml de tétrahydrofurane, on coule goutte à goutte, sous atmosphère d'azote, 0,1 mole de 4-(benzodioxan-5-yl)-1-[(3-chlorobenzocyclobutan-1-yl)carbonyl] pipérazine (R,S), préparée comme ci-dessus décrit, dans 100 ml de tétrahydrofurane. On laisse, une nuit, sous agitation, à température ambiante. On décompose, au bain de glace, par $H_2O$ : 2,6 ml, NaOH à 20 % : 2,1 ml et $H_2O$ : 9,5 ml.

On filtre le précipité, évapore le filtrat. On chromatographie l'huile résiduelle sur silice fine en éluant avec le système : $CH_2Cl_2-CH_3OH$ (95-5) pour obtenir la 4-(benzodioxan-5-yl)-1-[(3-chlorobenzocyclobutan-1-yl)méthyl] pipérazine (R,S), avec un rendement de 72 %.

- RMN (solvant : $CDCl_3$) :
  3H, 7,2 à 6,95 ppm (m) ; 1H, 6,8 ppm (t) ; 2H, 6,55 ppm (m) ;
  4H, 4,25 ppm (m) ; 1H, 3,7 ppm (m) ; 2H, 3,5 à 3,25 ppm (m) ;
  4H, 3,1 ppm (m) ; 6H, 3 à 2,7 ppm (m).

Une solution de 0,05 mole de la base ainsi obtenue dans 20 ml d'éther est maintenue sous agitation pendant 15 minutes avec 10 ml d'acide chlorhydrique normal. Puis on filtre, rince à l'éther et recristallise de l'eau le chlorhydrate de 4-(benzodioxan-5-yl)-1-[(3-chlorobenzocyclobutan-1-yl)méthyl] pipérazine (R,S), P.F. (K) : > 260 °C avec sublimation. (Rendement : 30 %).

**Exemples 12 à 29** :

En opérant comme décrit dans l'exemple 11, ont été préparés les dérivés objets des exemples ci-après :

**12)** La 4-(benzodioxan-5-yl)-1-[(3-fluorobenzocyclobutan-1-yl)méthyl] pipérazine (R,S) et son chlorhydrate P.F. (K) : 254-256 °C avec sublimation, par réduction de la 4(benzodioxan-5-yl)-1-[(3-fluorobenzocyclobutan-1-yl)carbonyl] pipérazine (R,S), (Rendement : 48 %), elle-même preparée avec un rendement de 40 %, à partir de l'acide (3-fluorobenzocyclobutan-1-yl)carboxylique [lui-même préparé selon la méthode décrite dans Tetrahedron (1974), 30, 1053, à partir de la 3-fluorobenzaldéhyde] et de la N-(benzodioxan-5-yl)pipérazine.

**13)** La 4-(benzodioxan-5-yl)-1-[3-(benzocyclobutan-1-yl)propyl] pipérazine (R,S) et son chlorhydrate P.F. (K) : 206-208 °C, par réduction de la 4-(benzodioxan-5-yl)-1-[3-(benzocyclobutan-1-yl) propionyl] pipérazine (Rendement : 65 %), elle-même preparée avec un rendement de 37 %, à partir de l'acide 3-(benzocyclobutan-1-yl)propionique (décrit dans la demande de brevet européen déposée par la demanderesse sous le n° 90.403145.7) et de la N-(benzodioxan-5-yl)-pipérazine.

**14)** La 4-(benzodioxan-5-yl)-1-[(indan-2-yl)méthyl] pipérazine et son chlorhydrate P.F. (K) : 232-234 °C, par réduction de la 4-(benzodioxan-5-yl)-1-[(indan-2-yl)carbonyl] pipérazine, P.F. (K) : 160-162 °C (Rendement : 43 %), elle-même preparée avec un rendement de 37 %, à partir de l'acide (indan-2-yl)-carboxylique [décrit dans J.A.C.S. (1975), 97 vol. 2, 347-353] et de la N-(benzodioxan-5-yl) pipérazine.

**15)** La 4-(benzodioxan-5-yl)-1-[(indan-1-yl)méthyl] pipérazine (H,S), P.F. (MK) : 87-90 °C, par réduction de la 4-(benzodioxan-5-yl)-1-[(indan-1-yl)carbonyl] pipérazine (Rendement : 70 %), elle-même preparée avec un rendement de 41 %, à partir de l'acide (indan-1-yl) carboxylique [décrit dans Synthesis (1987), 845] et de la N-(benzodioxan-5-yl)pipérazine.

**16)** La 4-(benzodioxan-5-yl)-1-[2-(5-méthoxybenzocyclobutan-1-yl)éthyl] pipérazine (R,S) (produit huileux) et son chlorhydrate P.F. (K) : 192-194 °C, par réduction de la 4-(benzodioxan-5-yl)-1-[2-(5-méthoxybenzocyclobutan-1-yl)acétyl] pipérazine (produit huileux) (Rendement : 62 %), elle-même preparée, avec un rendement de 72 %, à partir de l'acide 2-(5-méthoxybenzocyclobutan-1-yl)acétique, et de la N-(benzodioxan-5-yl)pipérazine.

L'acide 2-(5-méthoxybenzocyclobutan-1-yl)acétique a été préparé selon la méthode décrite dans J.A.C.S. (1975), 347, avec un rendement de 54 %, à partir du nitrile correspondant, lequel est obtenu, avec un rendement de 97 % à partir du tosylate correspondant, lui-même préparé, avec un rendement de 76 %, à partir de l'alcool correspondant et de paratoluène sulfochlorure, en milieu pyridine.

**17)** La 4-(benzodioxan-5-yl)-1-[2-(4,5-diméthoxybenzocyclobutan-1-yl)éthyl] pipérazine (R,S), (huile) et son chlorhydrate P.F. (K) : 232-234 °C, par réduction de la 4-(benzodioxan-5-yl)-1-[2-(4,5-diméthoxybenzocyclobutan-1-yl)acétyl] pipérazine (huile), (Rendement : 59 %), elle-même preparée, avec un rendement de 51,5 %, à partir de l'acide 2-(4,5-diméthoxybenzocyclobutan-1-yl)acétique, et de la N-(benzodioxan-5-yl)pipérazine.

L'acide 2-(4,5-diméthoxybenzocyclobutan-1-yl) acétique, P.F. (K) : 136-139 °C, a lui-même été obtenu, avec un rendement de 96 %, selon la méthode décrite dans J.A.C.S. (1975), 347, à partir du nitric correspondant, P.F. (K) : 110-112 °C.

**18)** La 4-(benzodioxan-5-yl)-1-[2-(ind-1-én-1-yl)éthyl]pipérazine et son chlorhydrate P.F (K) : 254-256 °C, par réduction de la 4-(benzodioxan-5-yl)-1-[2-(ind-1-én-1-yl)acétyl]pipérazine (Rendemnt : 30 %) elle-même preparée avec un rendement de 66 %, à partir de l'acide 2-(ind-1-én-1-yl)acétique [P.F (K) : 92-94 °C] et de la N-(benzodioxan-5-yl)pipérazine.

L'acide 2-(ind-1-én-1-yl)acétique a été préparé selon la méthode de H. Ahmed et N. Campbell J.C.S. (1960), 4115-4120, avec un rendement de 90 %, à partir du 2-(indan-1-ylidène)acétate d'éthyle, lui-même préparé avec un rendement de 48 % à partir d'indan-1-one et de $(C_6H_5)_3$ P=CH-COOC$_2$H$_5$ dans le toluène.

**19)** La 4-(benzodioxan-5-yl)-1-[2-(5,6-diméthoxyindan-1-yl)éthyl] pipérazine (R,S) et son chlorhydrate P.F (K) : 225-226 °C (méthanol), par réduction de la 4-(benzodioxan-5-yl)-1-[2-(5,6-diméthoxyindan-1-yl)-acétyl]pipérazine (Rendement : 25 %), elle-même preparée, avec un rendement de 98 %, à partir de l'acide 2-(5,6-diméthoxyindan-1-yl)acétique, P.F (K) : 151-153 °C, et de la N-(benzodioxan-5-yl)-pipérazine.

L'acide 2-(5,6-diméthoxyindan-1-yl)acétique a été préparé avec un rendement de 79 % à partir de l'ester éthylique correspondant (huile) lequel a été obtenu avec un rendement de 97 % à partir du 2-(5,6-diméthoxyindan-1-ylidène)acétate d'éthyle, lui-même préparé avec un rendement de 25 % à partir de 5,6-diméthoxyindan-1-one et de $(C_6H_5)_3$ P=CH-COOC$_2$H$_5$ dans le toluène.

**20)** La 4-(benzodioxan-5-yl)-1-[2-(indan-2-yl)éthyl]pipérazine P.F (ME) : 121-123 °C, par réduction de la 4-(benzodioxan-5-yl)-1-[2-(indan-2-yl)acétyl]pipérazine (huile) (Rendement : 64 %) elle-même préparée, avec un rendement de 90 % à partir de l'acide 2-(indan-2-yl)acétique P.F (M.K) : 91-93 °C et de la N-(benzodioxan-5-yl)pipérazine.

L'acide 2-(indan-2-yl)acétique a été préparé à partir de l'ester éthylique correspondant (huile) lequel a été obtenu avec un rendement de 98 % par hydrogénation du 2-(indan-2-ylidène)acétate d'éthyle (huile), lui-même préparé avec un rendement de 74 % à partir d'indan-2-one et de $(C_6H_5)_3$ P=CH-$COOC_2H_5$ dans le toluène.

**21)** La 4-(benzofuran-7-yl)-1-[3-(benzocyclobutan-1-yl)propyl]pipérazine (R,S) et son fumarate P.F (M.K) : 197-200 °C (méthanol), par réduction de la 4-(benzofuran-7-yl)-1-[3-(benzocyclobutan-1-yl)propionyl]-pipérazine (huile) (Rendement : 47 %), elle-même preparée, avec un rendement de 57 %, à partir de l'acide 3-(benzocyclobutan-1-yl)propionique et de la N-(benzofuran-7-yl)pipérazine, préparée selon J. Med. Chem. (1988), 31, 1934-1940.

**22)** La 4-(benzodioxan-5-yl)-1-[2-(indan-1-yl)éthyl]pipérazine (R,S) et son chlorhydrate P.F (K) : 220-222 °C, par réduction de la 4-(benzodioxan-5-yl) 1-[2-(indan-1-yl)acétyl]pipérazine (Rendement : 44 %), elle-même preparée, avec un rendement de 75 %, à partir de l'acide 2-(indan-1-yl)acétique et de la N-(benzodioxan-5-yl)pipérazine.

**23)** La 4-(benzodioxan-5-yl)-1-[3-(indan-1-yl)propyl]pipérazine (R,S) et son dichlorhydrate P.F (K) : 175-185 °C, par réduction de la 4-(benzodioxan-5-yl)-1-[3-indan-1-yl)propionyl]pipérazine (huile) (rendement : 71,5 %), elle-même preparée, avec un rendement de 85 %, à partir de l'acide 3-(indan-1-yl)propionique (huile) et de la N-(benzodioxan-5-yl)pipérazine.

L'acide 3-(indan-1-yl)propionique a été préparé comme suit : 27 g d'ester méthylique de l'acide 1-indane carboxylique [obtenu selon la méthode de F.M Nongrun et B. Myrboh, Synthesis (1987) 9, 845-846], dans 200 ml d'hydroxyde de sodium et 200 ml d'éthanol, sont agités une nuit à température ambiante. Après acidification à l'acide chlorhydrique concentré, on obtient 8 g d'acide 1-indane carboxylique P.F (K) 65 °C (Rendement : 30 %).

8 g de l'acide ainsi obtenu dans 200 ml de tétrahydrofurane sont ajoutés à une suspension de 1,55 g d'hydrure de lithium aluminium dans 40 ml de tétrahydrofurane et agités une nuit à température ambiante. Après hydrolyse par 1,07 ml d'eau puis 0,86 ml d'hydroxyde de sodium à 20 %, et enfin 4 ml d'eau, évaporation du solvant, le résidu est distillé au Kügelrohr. On obtient 4,3 g de 1-indane méthanol (huile-Eb/0.05 mmHg : 70-75 °C) (Rendement : 58 %).

10 g de cet alcool et 19 g de p-toluène sulfochlorure sont agités dans 80 ml de pyridine pendant 18 heures. Après évaporation du solvant, le milieu est lavé à l'eau et extrait à $CH_2Cl_2$. On obtient 14 g de tosylate de 1-indane méthanol sous forme d'huile, avec un rendement de 70 %.

5 g du tosylate ainsi obtenu dissous dans 5 ml d'éthanol sont ajoutés au mélange de 3,2 g de malonate de diéthyle lui-même ajouté goutte à goutte à une solution d'éthylate de sodium obtenue à partir de 0,46 g de sodium dans 10 ml d'éthanol. Le milieu réactionnel est alors amené et maintenu au reflux pendant 18 heures. Après dilution à l'acide chlorhydrique, on extrait le produit à l'acétate d'éthyle et le purifie sur colonne de silice en éluant avec $CH_2Cl_2$/cyclohexane (40/60). On obtient ainsi, avec un rendement de 56 %, l'ester éthylique de l'acide 3-(indan-1-yl)-2-éthoxycarbonyl propionique.

On porte à reflux 2,3 g de cet ester dans 5 ml d'eau et 2,5 g d'hydroxyde de potassium pendant 2 heures. Après acidification par HCl, on obtient 1,8 g d'acide 3-(indan-1-yl)-2-carboxy propionique, P.F (K) : 150-152 °C, (Rendement : 96 %).

1,8 g de ce diacide ainsi obtenu sont portés à reflux dans la N,N-diméthylacétamide pendant 2 heures 30. On obtient, après dilution à l'eau et extraction à l'éther, 1,3 g d'acide 3-(indan-1-yl)propionique sous forme d'huile avec un rendement de 89 %.

**24)** La 4-[benzo(1,5)dioxépin-6-yl]-1-[3-(benzocyclobutan-1-yl)propyl] pipérazine (R,S) et son chlorhydrate P.F (M.K) : 262-265 °C, par réduction de la 4 - [ benzo (1,5) dioxépin - 6 - yl] - 1 - [3 - (benzocyclobutan -1-yl)propionyl]pipérazine (huile) (Rendement : 37 %), elle-même preparée avec un rendement de 30 % à partir de l'acide 3-(benzocyclobutan-1-yl)propionique et de la N-[benzo(1,5)-dioxépin-6-yl]pipérazine.

**25)** La 4-(benzodioxan-5-yl)-1-[3-(indan-2-yl)propyl]pipérazine et son chlorhydrate P.F (K) : 210 °C, par réduction de la 4-(benzodioxan-5-yl)-1-[3-(indan-2-yl)propionyl]pipérazine (Rendement : 50 %), elle-même préparée avec un rendement de 86 % à partir de l'acide 3-(indan-2-yl)propionique, P.F (K) : 75-78 °C, et de la N-(benzodioxan-5-yl) pipérazine.

L'acide 3-(indan-2-yl)propionique a été préparé avec un rendement de 68 % à partir de l'acide 3-(indan-2-yl)-2-carboxy propionique, lequel a été préparé, avec un rendement de 44 %, à partir du diéthyl ester correspondant, qui est lui-même obtenu, avec un rendement de 69 %, à partir du mésylate d'

(indan-2-yl)éthyle et du di(éthoxycarbonyl )méthane.

**26)** La 4-(benzodioxan-5-yl)-1-[3-(3-chlorobenzocyclobutan-1-yl) propyl] pipérazine (R,S) et son dichlorhydrate P.F (M.K) : 223-226 °C, par réduction de la 4-(benzodioxan-5-yl)-1-[3-(3-chlorobenzocyclobutan-1-yl)propionyl ]pipérazine P.F (K) : 135-140 °C (Rendement : 84 %) elle-même preparée, avec un rendement de 74 %, à partir de l'acide 3-(3-chlorobenzocyclobutan-1-yl)propionique (huile) et de la N-(benzodiozan-5-yl)pipérazine.

L'acide 3-(3-chlorobenzocyclobutan-1-yl)propionique a été préparé, avec un rendement de 61 %, à partir de l'acide 3-(3-chlorobenzocyclobutan-1-yl)-2-carboxy propionique P.F (K) : 190-192 °C, lequel a été préparé, avec un rendement de 100 %, à partir du diéthylester correspondant lui-même obtenu, avec un rendement de 30 %, à partir du tosylate de (3-chlorobenzocyclobutan-1-yl)méthyle et du di-(éthoxycarbonyl)méthane.

**27)** La 4-(benzodioxan-5-yl)-1-[2-(1,2,3,4-tétrahydronaphtalén-1-yl)éthyl] pipérazine (R,S) et son chlorhydrate P.F instantané : 250-252 °C (acétonitrile) par réduction de la 4-(benzodioxan-5-yl)-1-[2-(1,2,3,4-tétrahydronaphtalén-1-yl)acétyl]pipérazine elle-même preparée à partir de l'acide (1,2,3,4-tétrahydronaphtalén-1-yl)acétique et de la N-(benzodioxan-5-yl)pipérazine.

L'acide (1,2,3,4-tétrahydronaphtalén-1-yl)acétique a lui-même été préparé à partir du 1-éthoxycarbonylméthyl 1,2,3,4-tétrahydronaphtalène lequel est obtenu à partir du 1-éthoxycarbonylméthyl 3,4-dihydronaphtalène [cf. J. Chem. Soc. (1960), 4115-4120], lui-même préparé à partir de 1,2,3,4-tétrahydronaphtalén-1-one.

**28)** La 4-(benzodioxan-5-yl)-1-[2-(benzocycloheptan-1-yl)éthyl]pipérazine (R,S) et son dichlorhydrate P.F (M.K) : 179-186 °C, par réduction de la 4-(benzodioxan-5-yl)-1-(2-(benzocycloheptan-1-yl)acétyl]-pipérazine, elle-même preparée à partir de l'acide (benzocycloheptan-1-yl)acétique et de la N-(benzodioxan-5-yl)pipérazine.

L'acide (benzocycloheptan-1-yl)acétique a été préparé à partir de 1-éthoxycarbonylméthyl benzocycloheptane, lui-même préparé à partir de benzocycloheptan-1-one.

**29)** La 4-(benzodioxan-5-yl)-1-[2-(benzocyclohept-1-én-1-yl)éthyl] pipérazine et son chlorhydrate P.F (M.K) : 233-236 °C par réduction de la 4-(benzodioxan-5-yl)-1-[(benzocyclohept-1-én-1-yl)acétyl]-pipérazine, elle-même preparée à partir de l'acide (benzocyclohept-1-én-1-yl)acétique et de la N-(benzodioxan-5-yl)pipérazine.

L'acide (benzocyclohept-1-én-1-yl)acétique a été préparé à partir du 1-éthoxycarbonylméthyl benzocyclohept-1-ène, lui-même préparé à partir de benzocycloheptan-1-one traité par

$$(C_2H_5O)_2-\overset{\overset{\large O}{\|}}{P}-CH_2-COOC_2H_5$$

et NaH dans le tétrahydrofurane, puis séparation des deux isomères exocycliques insaturés cis et trans également formés, en opérant par chromato-flash sur silice en éluant avec le toluène.

**Exemple 30 :**

ETUDE PHARMACOLOGIQUE

Les dérivés de la présente invention ont été étudiés comparativement à la Buspirone, produit de référence connu à titre de ligand des récepteurs sérotoninergiques 5 $HT_{1A}$.

**A) Méthodologie :**

Les essais ont été réalisés sur des rats Wistar mâles de 200 à 220 g ayant libre accès à leur nourriture et à leur eau de boisson, dans des cages standards.

Les animaux sont isolés individuellement pour les essais d'hypothermie, de secrétion de corticostérone et de position affaissée du corps (Flat Body Posture) ou réunis par goupes de trois pour le test des battements de la queue (Tail-Flicks).

La température du laboratoire est maintenue à 21 ± 1 °C sous une humidité de 60 ± 5 %. Ils sont soumis à un cycle lumière/obscurité de 12 heures/12 heures (le cycle lumière commençant à 7 h 30 du matin).

1) Etude in vitro - Test de Binding;

L'hippocampe issu des cerveaux de rats décapités a été immédiatement congélé sur glace carbonique puis conservé à - 80 °C jusqu'à la préparation des membranes. Le tissus a été homogénéisé à 4 °C dans le tampon approprié en utilisant un Polytron (Instruments Brinkman-Lucerne - Suisse) et centrifugé à 20.000 tours/mn.

L'incubation a été faite à 25 °C pendant 30 mn. La liaison non spécifique a été définie par 10 μmole de 5 HT. Les essais ont été terminés par filtration rapide à l'aide d'un collecteur de Brandel sur des filtres en fibre de verre prétraités avec 0,1 % de polyéthylène imine.

Pour chaque ligand froid, on a pris en compte un minimum de 3 valeurs produisant une inhibition entre 20 et 80 % de la liaison du ligand chaud. Les valeurs de concentrations inhibitrices 50 ($IC_{50}$) ont été déterminées selon le procédé 8 de Tallarida R.J et Murray R.B., Manual of Pharmacological calculations with computer programs, Springer Cerlag, New York, (1987).

Le pKi a été calculé selon la formule :

$$pKi = -\log\left(\frac{IC_{50}}{1+[L]/Kd}\right)$$

dans laquelle [L] est la concentration du ligand chaud ([13]H-8-OH-DPAT, 0,4 nM) et Kd est la constante de dissociation apparente déterminée à partir des expériences de saturation.

Les substances étudiées ont été solubilisées dans le tampon d'incubation.

2) Etude in vivo

a/ Processus général concernant les tests d'activités agonistes et antagonistes sur les récepteurs $5HT_{1A}$.

Les composés à étudier ont été administrés par voie sous-cutanée (s.c) 60 minutes avant le début du test c'est à dire 30 minutes avant le solvant (réponses agonistes) ou le 8-OH-DPAT (réponses antagonistes).

Dans tous les essais le solvant est utilisé en parallèle comme contrôle. Les animaux ont été laissés au repos dans leur cage pendant le temps compris entre les injections et l'évaluation. Pour les études agonistes, le solvant a été administré à 1 ml/kg s.c. 30 minutes avant le début du test. Pour les études antagonistes, on a choisi des doses de 8-OH-DPAT induisant des réponses sous-maximales soit des doses de 0,63-0,16-0,16 et 0,16 mg/kg s.c. respectivement pour les tests de Tail-Flicks, Flat Body Posture, Sécrétion de Corticostérone et d'Hypothermie.

b/ Position affaissée du corps (Flat Body Posture ou FBP) et Sécrétion de Corticostérone (CS).

Les mêmes animaux ont été employés pour évaluer l'influence des composés étudiés sur le FBP et sur la détermination de la concentration plasmatique de CS. Tous les essais ont été réalisés le matin entre 10 h 30 et 12 h 30, soit lorsque les taux circadien de CS sont les plus faibles.

25 minutes après le traitement (soit 5 minutes avant la décapitation) les animaux sont observés dans leurs cages et on note la présence ou non de FBP.

La présence de FBP est définie par une position caractéristique de l'animal. Celui-ci est alors en position de decubitus ventral avec les membres postérieurs nettement en extension. 5 minutes après l'observation de FBP, les animaux sont décapités et le sang du tronc est recueilli dans des tubes refroidis contenant 50 μl d'une solution de EDTA à 10 % . Après centrifugation à 4000 tours/mn, le plasma est prélevé et conservé à - 30 °C jusqu'au dosage.

La CS a été déterminée en utilisant un dosage radio-compétitif pour une protéine plasmatique fixant la CS : la trancortine. Celle-ci est obtenue à partir d'un sérum de singe. La séparation des complexes CS-transcortine de la CS libre a été réalisée au moyen d'une solution de Dextran et de charbon actif. La limite de détection était de 50 pg/tube. Les variations de dosage intra- et Inter-expériences étaient respectivement de 5 et 15 % [cf Rivet J.M. et al, Eur. J. Pharmacol., 183, 634-635 (1990)].

Les taux de base de CS dans le plasma n'étant jamais zéro, on a utilisé, pour calculer le pourcentage d'inhibition de CS plasmatique induite par 8-OH-DPAT, la formule suivante :

$$\%\ \text{d'inhibition} = 100 \times \frac{(\text{Antagoniste+Agoniste}) - \text{Antagoniste seul}}{(\text{Solvant+Agoniste}) - \text{Solvant seul}}$$

c/ Température corporelle (CT)

Les rats sont immobilisés et un thermomètre digital lubrifié (Thermistoprobe de Testotherm, Bale, suisse) est inséré dans le rectum à une profondeur de 5 cm. 30 secondes après l'insertion, la température est lue sur une échelle digitale. Le pourcentage d'inhibition est calculé à l'aide de la formule citée précédemment.

d/ Test spontané de Tail Flicks : (STF)

Les battements de la queue on été déterminés sur des animaux maintenus dans des cylindres en plastique opaque horizontaux, la queue des animaux pendant librement sur le bord de la paillasse de laboratoire. Après 5 minutes d'adaptation, on enregistre le nombre de mouvements émis en 5 minutes. Un STF est défini comme étant une élévation de la queue à un niveau supérieur à celui de l'axe du corps [Millan M.J. et al., J. Pharmacol. Exp. Ther., 256, 973-982 (1990)].

e/ Analyse des résultats in vivo

- En géneral, après analyse de variance, les résultats sont soumis au test de Dunett. Les résultats sont tenus pour significatifs si $p<0{,}05$.
- Pour l'analyse des courbes dose-réponse concernant l'induction des STF, CS et Hypothermie, on a déterminé la dose efficace minimale (M.E.D) en mg/kg, c'est à dire la dose qui induit une réponse significativement différente de celle produite par le solvant.
- Pour l'analyse des courbes dose-réponse concernant l'inhibition de STF, CS, et Hypothermie, les valeurs $ID_{50}$ -en mg/kg (dose réduisant de 50 % l'action de 8-OH-DPAT) ont été calculées ainsi que les limites de confiance à 95 % en utilisant une méthode inspirée de la méthode de Finney (1964).
- Pour la dose-réponse d'induction et d'inhibition du FBP, les doses efficaces 50 ($ED_{50}$) (doses pour lesquelles 50 % des animaux montrent une réponse) ont été calculées par la méthode de Litchfield et Wilcoxon.

f/ Composés étudiés

Les doses des composés testés sont toutes exprimées en terme de base. Sauf mention contraire, tous les composés ont été dissous dans de l'eau stérile (additionnée si nécessaire de quelques gouttes d'acide lactique) et administrés à un volume de 1 ml/kg s.c.

**B) Résultats :**

Les résultats sont regroupés dans les tableaux 1 et 2 ci-après.

Tableau 1

Binding des récepteurs 5 $HT_{1A}$

| MOLECULE | AFFINITE (pKi) |
|---|---|
| Produit de référence BUSPIRONE | 7,93 |
| Exemple 1 | 8,74 |
| Exemple 2 | 9,21 |
| Exemple 3 | 8,94 |
| Exemple 4 | 8,85 |
| Exemple 5 | 8,65 |
| Exemple 7 | 8,75 |
| Exemple 8 | 8,80 |
| Exemple 9 | 8,42 |
| Exemple 12 | 8,75 |
| Exemple 13 | 9,35 |
| Exemple 14 | 8,47 |
| Exemple 15 | 8,70 |
| Exemple 16 | 8,85 |
| Exemple 17 | 8,46 |
| Exemple 18 | 9,09 |
| Exemple 19 | 8,83 |
| Exemple 20 | 9,18 |
| Exemple 21 | 8,55 |
| Exemple 22 | 8,80 |
| Exemple 23 | 9,21 |
| Exemple 24 | 9,10 |

**TABLEAU 2 : ACTIVITE (IN VIVO) AGONISTE ET ANTAGONISTE DES RECEPTEURS 5 $HT_{1A}$**

| MOLECULE | SPONTANEOUS TAIL-FLICKS | | FLAT BODY POSTURE $ED_{50}$ (95 % C.L) | | CORTICO-STERONE SECRETION | | HYPOTHERMIE | |
|---|---|---|---|---|---|---|---|---|
| | M.E.D. | $ID_{50}$ (95 % C.L) | REPONSE AGONISTE | ANTAGONISME DE 8-OH-DPAT | M.E.D. | $ID_{50}$ (95 % C.L) | M.E.D. | $ID_{50}$ (95 % C.L) |
| Produit de réf. Buspirone | > 10,0 | 3,71 (1,40-9,84) | 7,4 (2,16-25,57) | >10,0 | >2,5 | >10,0 | 2,5 | - |
| Exemple 1 | > 10,0 | 0,09 (0,02-0,32) | >10,0 | 0,71 (0,09-5,34) | >2,5 | <2,5 | 10,0 | 0,5 (0,2-1,24) |
| Exemple 2 | >10,0 | 0,085 (0,025-0,28) | >20,0 | 0,76 (0,23-2,58) | 10,0 | 0,66 (0,35-1,24) | 40,0 | 0,65 (0,36-1,14) |
| Exemple 3 | >10,0 | 0,32 (0,077-1,38) | >10,0 | 1,55 (0,76-3,17) | >10,0 | 2,15 (1,21-3,84) | >20,0 | 1,64 (0,83-3,25) |
| Exemple 7 | >10,0 | 0,74 (0,28-1,93) | >10,0 | 0,52 (0,18-1,48) | >10,0 | 2,5 | 20,0 | 1,60 (0,80-3,20) |
| Exemple 9 | >10,0 | 1,59 (0,77-3,3) | >2,5 | <2,5 | >2,5 | >2,5 | 5,0 | 4,36 (1,67-11,42) |
| Exemple 13 | >10,0 | 0,13 (0,054-0,32) | >10,0 | 0,57 (0,17-1,87) | 10,0 | 1,27 (0,59-2,73) | 10,0 | 0,21 (0,04-0,97) |
| Exemple 14 | | 0,19 (0,05-0,74) | >2,5 | ≥2,5 | >2,5 | >2,5 | >10,0 | <2,5 |
| Exemple 15 | | 0,085 (0,03-0,22) | >2,5 | ≥2,5 | >2,5 | >2,5 | 10,0 | 1,53 (0,57-4,06) |
| Exemple 20 | | 0,18 (0,03-1,03) | >2,5 | <2,5 | >2,5 | <2,5 | 10,0 | 0,58 (0,22-1,49) |
| Exemple 22 | >10,0 | 0,74 (0,28-1,91) | >10,0 | 1,03 (0,52-2,03) | >10,0 | 1,92 (1,08-3,43) | >40,0 | 1,19 (0,59-2,42) |
| Exemple 23 | | 0,56 (0,17-1,81) | 10,0 | 0,29 (0,06-1,55) | >2,5 | <2,5 | 5,0 | 0,40 (0,12-1,37) |

$ID_{50}$=Dose inhibitrice 50 ; $ED_{50}$=Dose efficace 50 ; CL=limite de confiance ; MED=Dose efficace minimale.

**C) Conclusion :**

L'examen des résultats repertoriés dans les tableaux 1 et 2 montre que les composés de la présente invention ont un comportement antagoniste des récepteurs $5HT_{1A}$ contrairement à la buspirone qui, bien que se fixant également sur les récepteurs 5HT1A, a un comportement agoniste.

D'où l'intérêt des composés de la présente invention dans le traitement des maladies du système nerveux central et des maladies neuroendocriniennes.

## Revendications

**Revendications pour les Etats contractants suivants : AT, BE, CH, DE, DK, FR, GB, IT, LI, LU, NL, SE**

**1.** Les pipérazines 1,4-disubstituées de formule générale I :

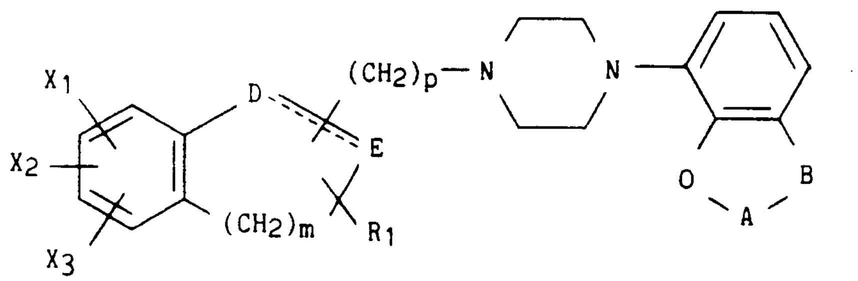

(I)

dans laquelle :

- $X_1$, $X_2$ et $X_3$ :
  - identiques ou différents, représentent chacun : un atome d'hydrogène ou d'halogène, un radical alkyle en chaine droite ou ramifiée contenant de 1 à 5 atomes de carbone, un radical hydroxy, un radical alcoxy ou alkylthio contenant chacun de 1 à 5 atomes de carbone en chaine droite ou ramifiée, un radical trifluorométhyle, un radical nitro, un radical amino, ou un radical acétamido, ou
  - deux d'entre eux pris en position adjacente forment ensemble un radical méthylènedioxy ou un radical éthylènedioxy ;
- $R_1$ représente un atome d'hydrogène ou un radical alkyle en chaine droite ou ramifiée contenant de 1 à 5 atomes de carbone ;
- - D=E - représente : $-(CH_2)_n-(CH_2)-$ ou $-CH=CH-$
- m et n représentent chacun les valeurs zéro, un, deux ou trois à condition que m + n soit ≧ 1.
- p représente zéro ou un nombre entier de 1 à 6, et
- -A-B- représente un des radicaux de formule :
  $-(CH_2)_2-O-$ ; $-(CH_2)_3-O-$ ; $-CH=CH-$ ; $-CH_2-CH_2-$ et

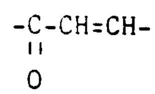 .

sous formes racémiques et optiquement actives.

**2.** Les sels physiologiquement tolérables des dérivés de la revendication 1 avec des acides appropriés.

**3.** La 4-(benzodioxan-5-yl)-1-(2-(benzocyclobutan-1-yl)éthylpipérazine (R,S) et son dichlorhydrate.

**4.** La 4-(benzodioxan-5-yl)-1-(2-(3-chlorobenzocyclobutan-1-yl)éthyl] pipérazine (R,S) et son dichlorhydrate.

**5.** La 4-(benzodioxan-5-yl)-1-(indan-2-yl)pipérazine.

**6.** La 4-(benzodioxan-5-yl)-1-(4-(benzocyclobutan-1-yl)butylpipérazine(R,S) et son fumarate.

**7.** La 4-[benzo (1,5) dioxépin-6-yl]-1-(indan-2-yl)pipérazine.

**8.** La 4-[benzodioxan-5-yl)-1-(2-(ind-1-én-1-yl)éthyl]pipérazine et son chlorhydrate.

**9.** La 4-[benzodioxan-5-yl)-1-(2-(indan-1-yl)éthyl]pipérazine (R,S) et son chlorhydrate.

**10.** Le procédé de préparation des dérivés de la revendication 1 caractérisé en ce que l'on condense :

- une pipérazine N-monosubstituée de formule générale II :

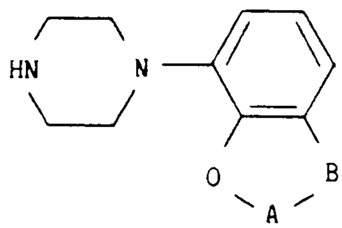

(II)

dans laquelle le groupe -A-B- a la signification définie dans la revendication 1,

- avec un dérivé de formule générale III :

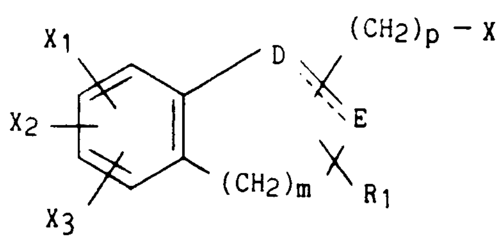

(III)

dans laquelle :

- $X_1$, $X_2$, $X_3$, $R_1$, - D$=$E-, m et p ont les significations définies dans la revendication 1, et
- X représente un atome d'halogène, ou un radical mésyloxy ou tosyloxy.

**11.** Le procédé de préparation selon la revendication 10 caractérisé en ce que l'on effectue la condensation des dérivés II et III dans un solvant approprié, à une température comprise entre 20 et 150 °C, en présence d'un accepteur de l'acide formé au cours de la réaction.

**12.** Le procédé de préparation des dérivés de la revendication 1 répondant à la formule générale I' :

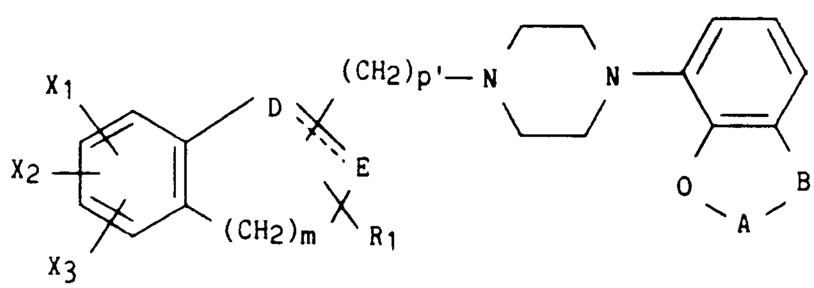

(I')

dans laquelle :

- $X_1$, $X_2$, $X_3$, $R_1$, - D$=$E - ,m et -A-B- ont les significations définies dans la revendication 1, et
- p' représente un nombre entier de 1 à 6.

caractérisé en ce que :

- l'on condense :
  - la pipérazine N-monosubsituée de formule générale II définie dans la revendication 10, avec

- un dérivé de formule générale IV :

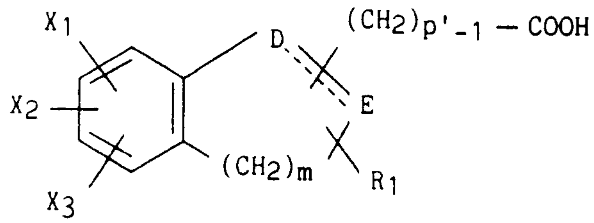

(IV)

dans laquelle :

- $X_1$, $X_2$, $X_3$, $R_1$ - D=E - , m et p' ont les significations précédemment définies ; et
- l'on réduit l'amide ainsi obtenue de formule générale V :

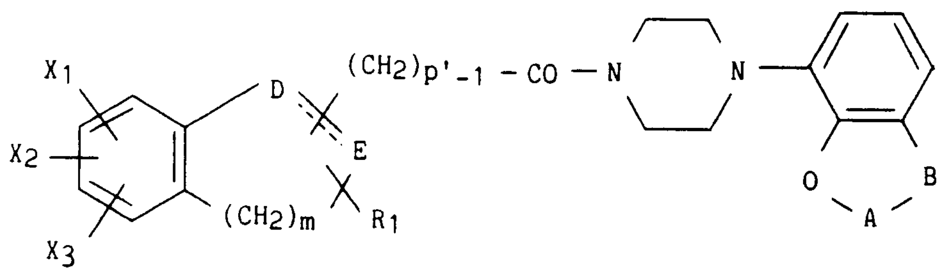

(V)

dans laquelle :

- $X_1$, $X_2$, $X_3$, $R_1$, - D=E - ,m, p' et -A-B- ont les significations précédemment définies .

**13.** Le procédé de préparation selon la revendication 12 caractérisé en ce que l'on effectue la condensation des dérivés II et IV dans le chlorure de méthylène en présence de carbonyldiimidazole.

**14.** Le procédé de préparation selon la revendication 12 caractérisé en ce que l'on effectue la réduction de l'amide V au moyen d'un hydrure double de lithium-aluminium dans un solvant approprie.

**15.** Les compositions pharmaceutiques contenant comme principe actif un dérivé selon une des revendications 1 à 9 avec des excipients pharmaceutiques appropriés.

**16.** Les compositions pharmaceutiques selon la revendication 15, présentées sous une forme convenant notamment pour le traitement des maladies du système nerveux central et des maladies neuroendocriniennes.

**17.** A titre de produits intermédiaires nouveaux utilisables dans la synthèse des dérivés I', les amides de formule générale V :

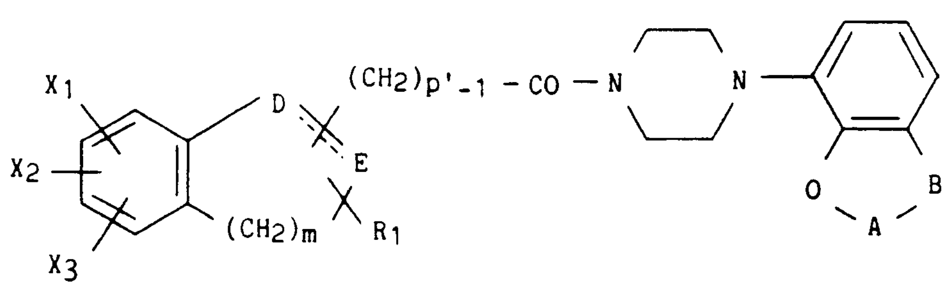

(V)

dans laquelle

- $X_1$, $X_2$, $X_3$, $R_1$, - D=E - , m, p' et -A-B- ont les significations définies dans la revendication 12.

**Revendications pour les Etats contractants suivants : ES, GR**

**1.** Le procédé de préparation des pipérazines 1,4-disubstituées de formule générale I :

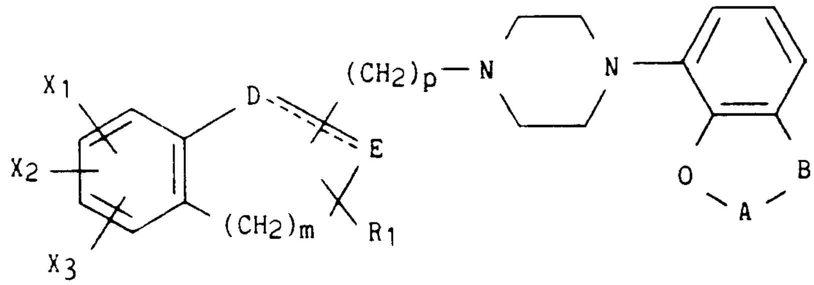

(I)

dans laquelle :

- $X_1$, $X_2$ et $X_3$ :
  - identiques ou différents, représentent chacun : un atome d'hydrogène ou d'halogène, un radical alkyle an chaine droite ou ramifiée contenant de 1 à 5 atomes de carbone, un radical hydroxy, un radical alcoxy ou alkylthio contenant chacun de 1 à 5 atomes de carbone en chaine droite ou ramifiée, un radical trifluorométhyle, un radical nitro, un radical amino, ou un radical acétamido, ou
  - deux d'entre eux pris en position adjacente forment ensemble un radical méthylènedioxy ou un radical éthylènedioxy ;
- $R_1$ représente un atome d'hydrogène ou un radical alkyle en chaine droite ou ramifiée contenant de 1 à 5 atomes de carbone ;
- - D$\equiv$E - représente : $-(CH_2)_n-(CH_2)-$ ou $-CH=CH-$
- m et n représentent chacun les valeurs zéro, un, deux ou trois à condition que m + n soit ≧ 1.
- p représente zéro ou un nombre entier de 1 à 6, et
- -A-B- représente un des radicaux de formule :
  $-(CH_2)_2-O-$ ; $-(CH_2)_3-O-$ ; $-CH=CH-$ ; $-CH_2-CH_2-$ et

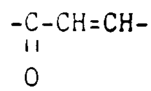 .

sous formes racémiques et optiquement actives,

ainsi que leurs sels physiologiquement tolérables avec des acides appropriés, caractérisé en ce que l'on condense :

- une pipérazine N-monosubstituée de formule générale II :

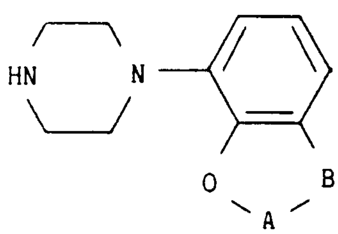

(II)

dans laquelle le groupe -A-B- a la signification précédemment définie ,

- avec un dérivé de formule générale III :

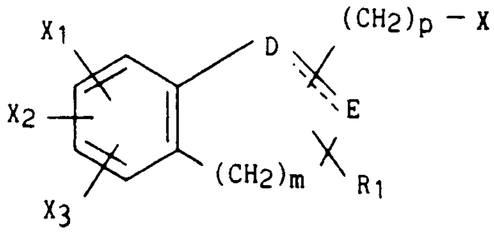

(III)

dans laquelle :

- $X_1$, $X_2$, $X_3$, $R_1$, - D⎓E -, m et p ont les significations précédemment définies , et
- X représente un atome d'halogène, ou un radical mésyloxy ou tosyloxy ;

- et si on le désire, on traite les dérivés I ainsi obtenus avec des acides appropriés pour donner les sels d'addition acides correspondants.

**2.** Le procédé de préparation selon la revendication 1 caractérisé en ce que l'on effectue la condensation des dérivés II et III dans un solvant approprié, à une temperature comprise entre 20 et 150 °C, en présence d'un accepteur de l'acide formé au cours de la réaction.

**3.** Le procédé de préparation des dérivés (I) répondant à la formule générale I' :

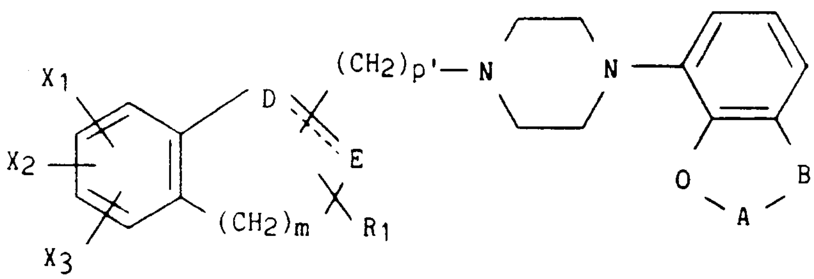

(I')

dans laquelle :

- $X_1$, $X_2$, $X_3$, $R_1$, - D⎓E - , m et -A-B- ont les significations définies dans la revendication 1, et
- p' représente un nombre entier de 1 à 6,

caractérisé en ce que :

- l'on condense :
  - la pipérazine N-monosubsituée de formule générale II définie dans la revendication 1, avec
  - un dérivé de formule générale IV :

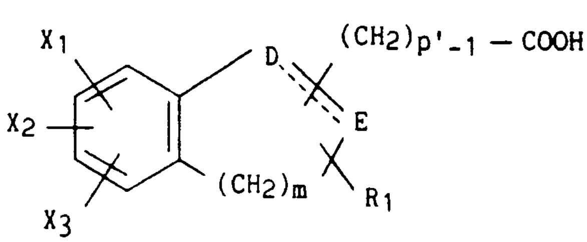

(IV)

dans laquelle :

- $X_1$, $X_2$, $X_3$, $R_1$, - D⎓E - , m et p' ont les significations précédemment définies ; et

- l'on réduit l'amide ainsi obtenue de formule générale V :

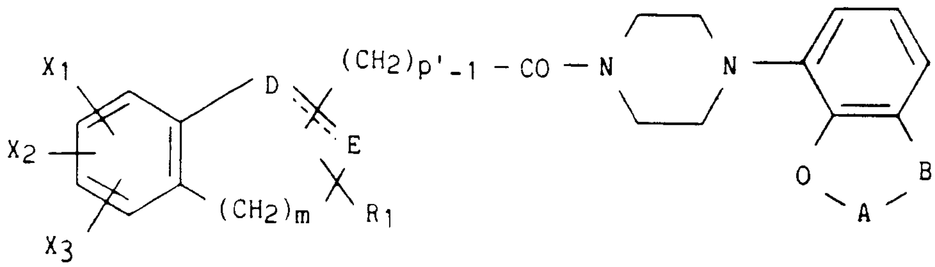

(V)

dans laquelle :

- $X_1$, $X_2$, $X_3$, $R_1$, - D=E - , m, p' et -A-B- ont les significations précédemment définies ;
- et si on le désire, on traite les dérivés I' ainsi obtenus avec des acides appropriés pour donner les sels d'addition acides correspondants.

**4.** Le procédé de préparation selon la revendication 3 caractérisé en ce que l'on effectue la condensation des dérivés II et IV dans le chlorure de méthylène en présence de carbonyldiimidazole.

**5.** Le procédé de préparation selon la revendication 3 caractérisé en ce que l'on effectue la réduction de l'amide V au moyen d'un hydrure double de lithium-aluminium dans un solvant approprié.

**6.** Le procédé de préparation des amides de formule générale V :

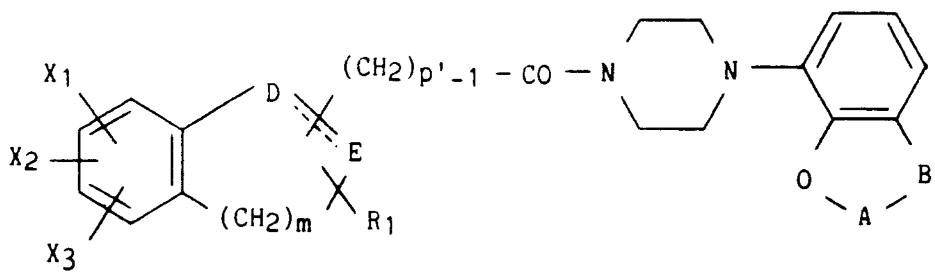

(V)

dans laquelle

- $X_1$, $X_2$, $X_3$, $R_1$, - D=E - , m, p' et -A-B- ont les significations définies dans la revendication 3,

caractérisé en ce que l'on condense les dérivés II et III comme inclus dans la revendication 3.

## Claims

**Claims for the following Contracting States : AT, BE, CH, DE, DK, FR, GB, IT, LI, LU, NL, SE**

**1.** 1,4-disubstituted piperazines of the general formula I:

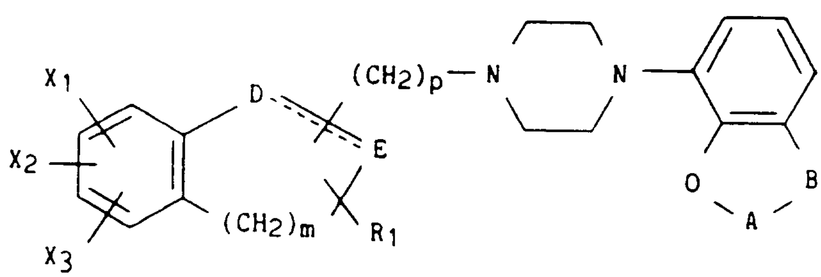

(I)

in which:

- $X_1$, $X_2$ and $X_3$,
  - which may be the same or different, each represent: a hydrogen or halogen atom, a straight-chained or branched-chained alkyl radical containing from 1 to 5 carbon atoms, a hydroxy

radical, a straight-chained or branched-chained alkoxy or alkylthio radical each containing from 1 to 5 carbon atoms, a trifluoromethyl radical, a nitro radical, an amino radical or an acetamido radical, or

- two of them in adjacent positions together form a methylenedioxy radical or an ethylenedioxy radical;

- $R_1$ represents a hydrogen atom or a straight-chained or branched-chained alkyl radical containing from 1 to 5 carbon atoms;
- -D=E- represents: $-(CH_2)_n-(CH_2)-X$ or -CH = CH-;
- each of m and n represents 0, 1, 2 or 3, with the proviso that m + n ≧ 1;
- p represents 0 or an integer from 1 to 6; and
- -A-B- represents a radical of the formula: $-(CH_2)_2-O-$;$-(CH_2)_3-O-$; -CH = CH-; $-CH_2-CH_2-$; or

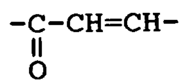

in racemic and optically active forms.

**2.** The physiologically tolerable salts of the compounds of claim 1 with suitable acids.

**3.** (R,S)-4-(benzodioxan-5-yl)-1-[2-(benzocyclobutan-1-yl)-ethyl]-piperazine and its dihydrochloride.

**4.** (R,S)-4-(benzodioxan-5-yl)-1-[2-(3-chlorobenzocyclobutan-1-yl)-ethyl]-piperazine and its dihydrochloride.

**5.** 4-(benzodioxan-5-yl)-1-(indan-2-yl)-piperazine.

**6.** (R,S)-4-(benzodioxan-5-yl)-1-[4-(benzocyclobutan-1-yl)-butyl]-piperazine and its fumarate.

**7.** 4-[benzo(1,5)dioxepin-6-yl]-1-(indan-2-yl)-piperazine.

**8.** 4-(benzodioxan-5-yl)-1-[2-(ind-1-en-1-yl)-ethyl]-piperazine and its hydrochloride.

**9.** (R,S)-4-(benzodioxan-5-yl)-1-[2-(indan-1-yl)-ethyl]-piperazine and its hydrochloride.

**10.** Process for the preparation of compounds of claim 1, characterised in that:

- an N-monosubstituted piperazine of the general formula II:

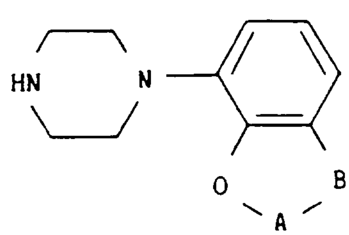

(II),

in which the group -A-B- has the meaning defined in claim 1, is condensed:

- with a compound of the general formula III:

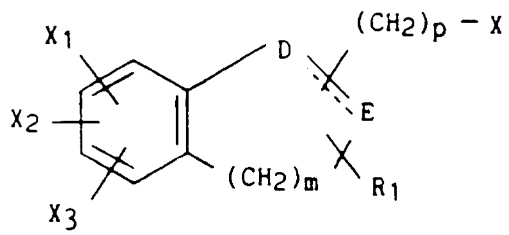

(III),

in which:
- $X_1$, $X_2$, $X_3$, $R_1$, -D⩦E-, m and p have the meanings defined in claim 1, and
- X represents a halogen atom, or a mesyloxy or tosyloxy radical.

**11.** Preparation process according to claim 10, characterised in that the condensation of the compounds II and III is carried out in a suitable solvent at a temperature of from 20 to 150 °C, in the presence of an acceptor for the acid formed during the reaction.

**12.** Process for the preparation of compounds of claim 1 corresponding to the general formula I':

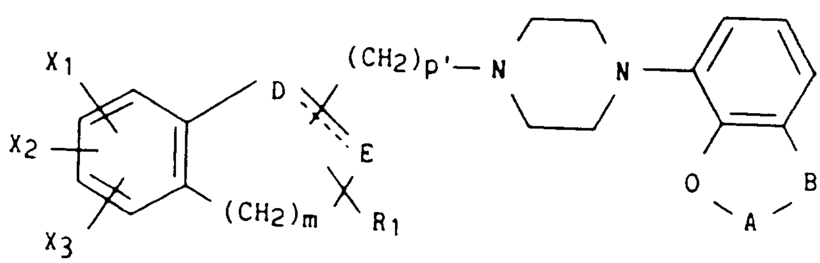

(I'),

in which:
- $X_1$, $X_2$, $X_3$, $R_1$, -D⩦E-, m and -A-B- have the meanings defined in claim 1, and
- p' represents an integer from 1 to 6,

characterised in that:
- the N-monosubstituted piperazine of the general formula II defined in claim 10 is condensed with:
- a compound of the general formula IV:

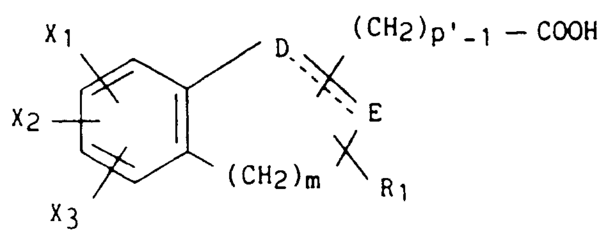

(IV),

in which:
- $X_1$, $X_2$, $X_3$, $R_1$, -D⩦E-, m and p' have the meanings defined above; and

- the resulting amide of the general formula V:

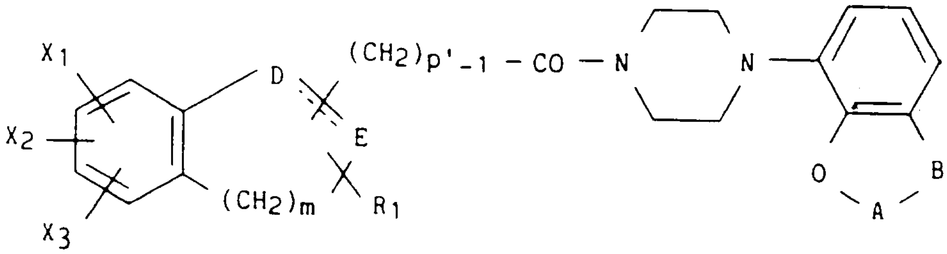

(V),

in which:

- $X_1$, $X_2$, $X_3$, $R_1$, -D$\overline{---}$E-, m, p' and -A-B- have the meanings defined above,

is reduced.

**13.** Preparation process according to claim 12, characterised in that the condensation of the compounds II and IV is carried out in methylene chloride in the presence of carbonyldiimidazole.

**14.** Preparation process according to claim 12, characterised in that the amide V is reduced by means of a double hydride of lithium and aluminium in a suitable solvent.

**15.** Pharmaceutical compositions containing as active ingredient a compound according to one of claims 1 to 9, together with suitable pharmaceutical excipients.

**16.** Pharmaceutical compositions according to claim 15 in a form suitable in particular for the treatment of disorders of the central nervous system and of neuroendocrine disorders.

**17.** As new intermediates that can be used in the synthesis of the compounds I', the amides of the general formula V:

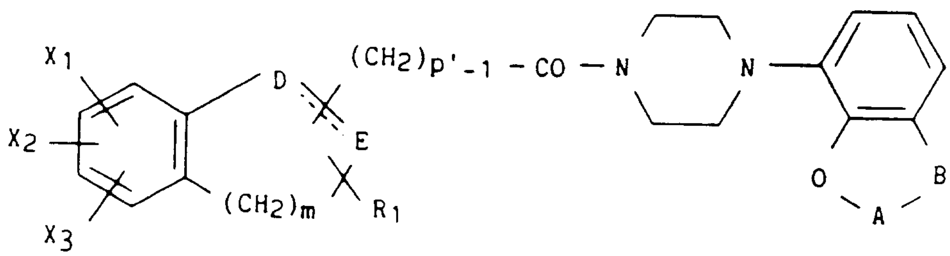

(V)

in which:

- $X_1$, $X_2$, $X_3$, $R_1$, -D$\overline{---}$E-, m, p' and -A-B- have the meanings defined in claim 12.

**Claims for the following Contracting States : ES, GR**

**1.** Process for the preparation of 1,4-disubstituted piperazines of the general formula I:

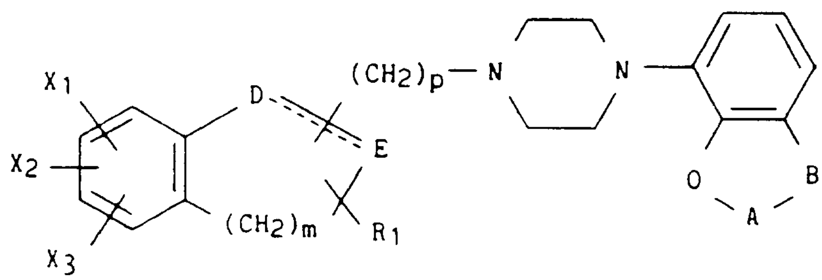

(I)

in which:

- $X_1$, $X_2$ and $X_3$,
  - which may be the same or different, each represent: a hydrogen or halogen atom, a straight-chained or branched-chained alkyl radical containing from 1 to 5 carbon atoms, a hydroxy radical, a straight-chained or branched-chained alkoxy or alkylthio radical each containing from 1 to 5 carbon atoms, a trifluoromethyl radical, a nitro radical, an amino radical or an acetamido radical, or
  - two of them in adjacent positions together form a methylenedioxy radical or an ethylenedioxy radical;
- $R_1$ represents a hydrogen atom or a straight-chained or branched-chained alkyl radical containing from 1 to 5 carbon atoms;
- -D$\overline{\overline{---}}$E- represents: $-(CH_2)_n-(CH_2)-$ or -CH = CH-;
- each of $\underline{m}$ and $\underline{n}$ represents 0, 1, 2 or 3, with the proviso that $\underline{m} + \underline{n} \geq 1$;
- $\underline{p}$ represents 0 or an integer from 1 to 6; and
- -A-B- represents a radical of the formula: $-(CH_2)_2-O-$;$-(CH_2)_3-O-$; -CH = CH-; $-CH_2-CH_2-$; or

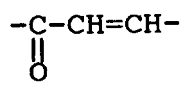 ,

in racemic and optically active forms,
and their physiologically tolerable salts with suitable acids,
which process is characterised in that:

- an N-monosubstituted piperazine of the general formula II:

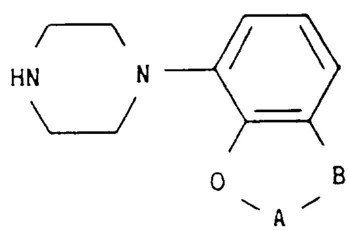

(II),

in which the group -A-B- has the meaning defined above,
is condensed:

- with a compound of the general formula III:

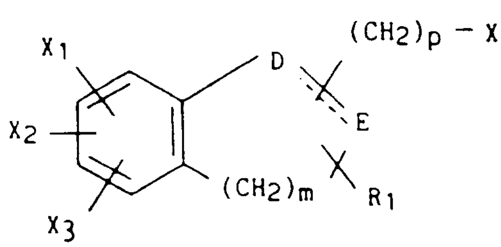

(III),

in which:

- $X_1$, $X_2$, $X_3$, $R_1$, -D$\overline{\overline{---}}$E-, $\underline{m}$ and $\underline{p}$ have the meanings defined above, and
- X represents a halogen atom, or a mesyloxy or tosyloxy radical;
- and, if desired, the resulting compounds I are treated with suitable acids to give the corresponding acid addition salts.

**2.** Preparation process according to claim 1, characterised in that the condensation of the compounds II and III is carried out in a suitable solvent at a temperature of from 20 to 150 °C, in the presence of an

acceptor for the acid formed during the reaction.

**3.** Process for the preparation of compounds (I) corresponding to the general formula I':

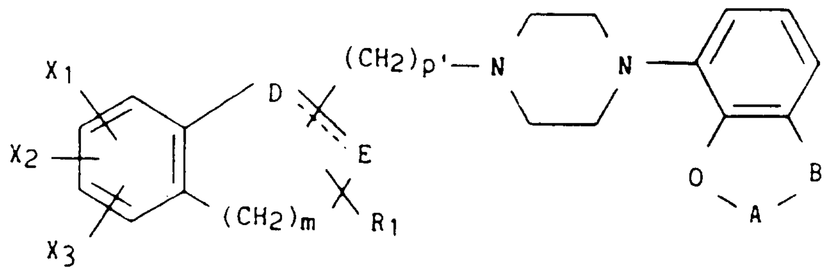

(I'),

in which:

- $X_1$, $X_2$, $X_3$, $R_1$, -D=E-, m and -A-B- have the meanings defined in claim 1, and
- p' represents an integer from 1 to 6,

characterised in that:

- the N-monosubstituted piperazine of the general formula II defined in claim 1 is condensed with:
- a compound of the general formula IV:

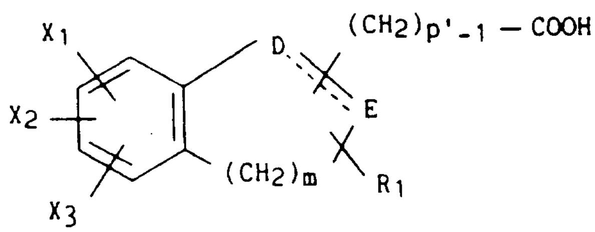

(IV),

in which:

- $X_1$, $X_2$, $X_3$, $R_1$, -D=E-, m and p' have the meanings defined above; and
- the resulting amide of the general formula V:

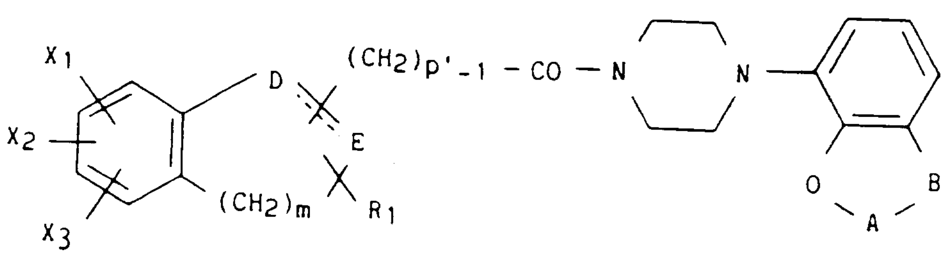

(V),

in which:

- $X_1$, $X_2$, $X_3$, $R_1$, -D=E-, m, p' and -A-B- have the meanings defined above,

is reduced;

- and, if desired, the resulting compounds I' are treated with suitable acids to give the corresponding acid addition salts.

**4.** Preparation process according to claim 3, characterised in that the condensation of the compounds II and IV is carried out in methylene chloride in the presence of carbonyldiimidazole.

**5.** Preparation process according to claim 3, characterised in that the amide V is reduced by means of a double hydride of lithium and aluminium in a suitable solvent.

**6.** Process for the preparation of amides of the general formula V:

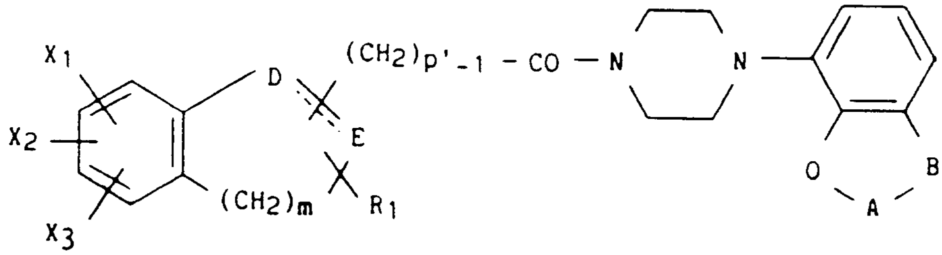

(V)

in which:

- $X_1$, $X_2$, $X_3$, $R_1$, -D=E-, m, p' and -A-B- have the meanings defined in claim 3,

characterised in that the compounds II and IV are condensed as included in claim 3.

**Patentansprüche**

**Patensprüche für folgende Vertragsstaaten : AT, BE, CH, DE, DK, FR, GB, IT, LI, LU, NL, SE**

**1.** 1,4-disubstituierte Piperazine der allgemeinen Formel I:

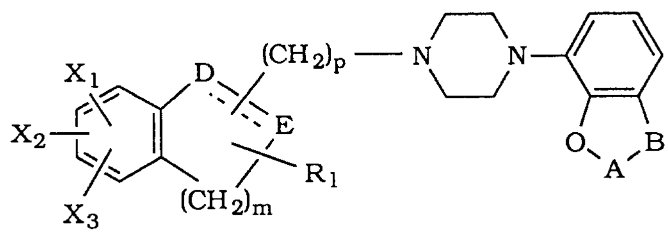

(I)

in der:

- $X_1$, $X_2$ und $X_3$:
  - die gleichartig oder verschieden sein können, jeweils ein Wasserstoff- oder Halogenatom, eine geradkettige oder verzweigte Alkylgruppe mit 1 bis 5 Kohlenstoffatomen, eine Hydroxylgruppe, eine Alkoxy- oder Alkylthiogruppe mit 1 bis 5 Kohlenstoffatomen in gerader oder verzweigter Kette, eine Trifluormethylgruppe, eine Nitrogruppe, eine Aminogruppe oder eine Acetamidogruppe, oder
  - jeweils zwei diese Gruppen in benachbarter Stellung gemeinsam eine Methylendioxy- oder Ethylendioxy-gruppe;
- $R_1$ eine Wasserstoffatom oder eine geradkettige oder verzweigte Alkylgruppe mit 1 bis 5 Kohlenstoffatomen;

  -D=E- eine Gruppe der Formel: $(CH_2)_n$-$(CH_2)$- oder -CH = CH-.
- m und n jeweils die Werte Null. Eins, Zwei oder Drei mit der Maßgabe besitzen, daß m + n $\geqq$ 1 ist,
- p Null oder eine ganze Zahl mit einem Wert von 1 bis 6, und
- -A-B- eine der Gruppen der Formel:

  -$(CH_2)_2$-O-; -$(CH_2)_3$-O-; -CH = CH-; -$CH_2$-$CH_2$- und

$$-\underset{\underset{O}{\|}}{C}-CH=CH-$$

bedeuten,

in racemischer oder optisch aktiver Form.

**2.** Die physiologisch verträglichen Salze der Derivate nach Anspruch 1 mit geeigneten Säuren.

**3.** 4-(Benzodioxan-5-yl)-1-[2-(benzocyclobutan-1-yl)-ethyl]-piperazin (R,S) und dessen Dihydrochlorid.

**4.** 4-(Benzodioxan-5-yl)-1-[2-(3-chlorbenzocyclobutan-1-yl)-ethyl]-piperazin (R,S) und dessen Dihydrochlorid.

**5.** 4-(Benzodioxan-5-yl)-1-(indan-2-yl)-piperazin.

**6.** 4-(Benzodioxan-5-yl)-1-[4-(benzocyclobutan-1-yl)-butyl]-piperazin (R,S) und dessen Fumarat.

**7.** 4-[Benzo(1,5)dioxepin-6-yl]-1-(indan-2-yl)-piperazin.

**8.** 4-(Benzodioxan-5-yl)-1-[2-(ind-1-en-1-yl)-ethyl]-piperazin und dessen Hydrochlorid.

**9.** 4-(Benzodioxan-5-yl)-1-[2-(indan-1-yl)-ethyl]-piperazin (R,S) und dessen Hydrochlorid.

**10.** Verfahren zur Herstellung der Derivate nach Anspruch 1, **dadurch gekennzeichnet**, daß man:
- ein N-monosubstituiertes Piperazin der allgemeinen Formel II:

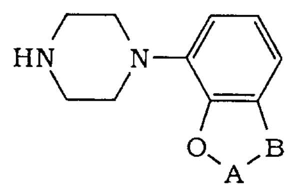

(II)

in der die Gruppe -A-B- die in Anspruch 1 angegebenen Bedeutungen besitzt,
- mit einem Derivat der allgemeinen Formel III:

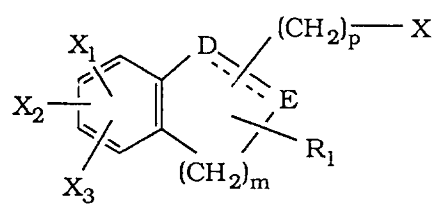

(III)

in der:
- $X_1$, $X_2$, $X_3$. $R_1$, - D$\overline{\cdots}$E- , m und p die in Anspruch 1 angegebenen Bedeutungen besitzen und
- X ein Halogenatom oder eine Mesyloxy- oder Tosyloxygruppe darstellt,

kondensiert.

**11.** Verfahren nach Anspruch 10, **dadurch gekennzeichnet**, daß man die Kondensation der Derivate II und III in einem geeigneten Lösungsmittel bei einer Temperatur zwischen 20 und 150 °C in Gegenwart eines Akzeptors für die im Verlaufe der Reaktion gebildete Säure durchführt.

**12.** Verfahren zur Herstellung der Derivate nach Anspruch 1 der allgemeinen Formel I':

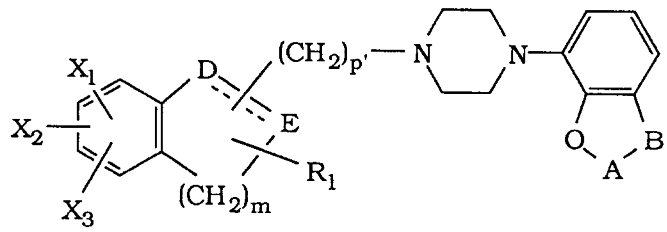

(I')

in der:

- $X_1$, $X_2$, $X_3$, $R_1$, -D==E-, m und -A-B- die in Anspruch 1 angegebenen Bedeutungen besitzen und
- p' eine ganze Zahl mit einem Wert von 1 bis 6 darstellt,

**dadurch gekennzeichnet**, daß man

- das N-monosubstituierte Piperazin der in Anspruch 10 definierten allgemeinen Formel II mit
- einem Derivat der allgemeinen Formel IV:

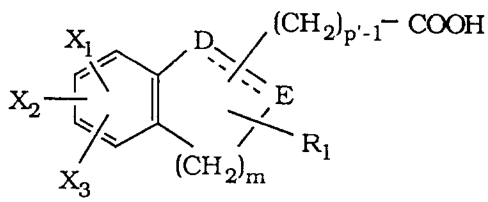

(IV)

in der:

- $X_1$, $X_2$, $X_3$, $R_1$, -D==E- , m und p' die oben angegebenen Bedeutungen besitzen, kondensiert; und
- das in dieser Weise erhaltene Amid der allgemeinen Formel V:

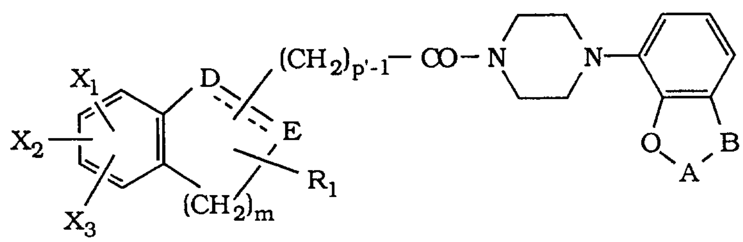

(V)

in der:

- $X_1$, $X_2$, $X_3$. $R_1$, -D==E- , m, p' und -A-B- die oben angegebenen Bedeutungen besitzen, reduziert.

**13.** Verfahren nach Anspruch 12, **dadurch gekennzeichnet**, daß man die Kondensation der Derivate II und IV in Methylenchlorid und in Gegenwart von Carbonyldiimidazol bewirkt.

**14.** Verfahren nach Anspruch 12, **dadurch gekennzeichnet**, daß man die Reduktion des Amids V mit einem Lithium-Aluminium-Doppelhydrid in einem geeigneten Lösungsmittel bewirkt.

**15.** Pharmazeutische Zubereitungen enthaltend als Wirkstoff ein Derivat nach einem der Ansprüche 1 bis 9 mit geeigneten pharmazeutischen Trägermaterialien.

**16.** Pharmazeutische Zubereitungen nach Anspruch 15 in einer Form, die insbesondere zur Behandlung von Erkrankungen des Zentralnervensystems und von neuroendokrinen Erkrankungen geeignet sind.

**17.** Als Zwischenprodukte für die Herstellung der Derivate I' geeignete Amide der allgemeinen Formel V:

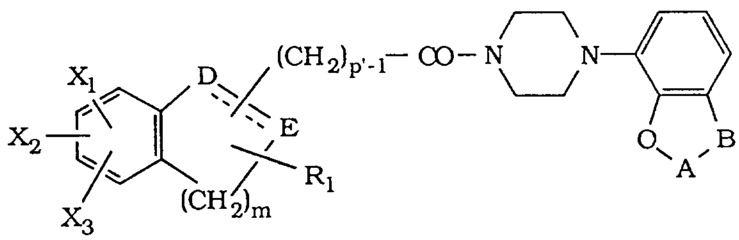

(V)

in der:

- $X_1$, $X_2$, $X_3$, $R_1$, -D═E- , m, p' und -A-B- die in Anspruch 12 angegebenen Bedeutungen besitzen.

**Patentansprüche für folgende Vertragsstaaten : ES, GR**

**1.** Verfahren zur Herstellung von 1,4-disubstituierten Piperazinen der allgemeinen Formel I:

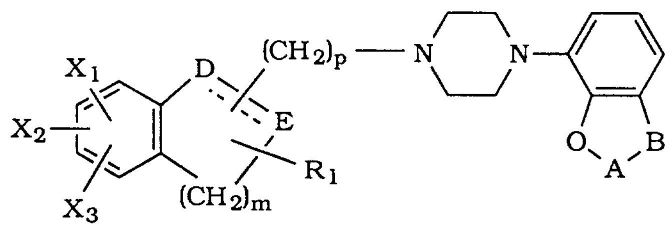

(I)

in der:

- $X_1$, $X_2$ und $X_3$:
  - die gleichartig oder verschieden sein können, jeweils ein Wasserstoff- oder Halogenatom, eine geradkettige oder verzweigte Alkylgruppe mit 1 bis 5 Kohlenstoffatomen, eine Hydroxylgruppe, eine Alkoxy- oder Alkylthiogruppe mit 1 bis 5 Kohlenstoffatomen in gerader oderverzweigter Kette, eine Trifluormethylgruppe, eine Nitrogruppe, eine Aminogruppe oder eine Acetamidogruppe, oder
  - jeweils zwei diese Gruppen in benachbarter Stellung gemeinsam eine Methylendioxy- oder Ethylendioxy-gruppe;
- $R_1$ eine Wasserstoffatom oder eine geradkettige oder verzweigte Alkylgruppe mit 1 bis 5 Kohlenstoffatomen;

  -D═E- eine Gruppe der Formel: $-(CH_2)_n-(CH_2)-$ oder $-CH=CH-$
- m und n jeweils die Werte Null, Eins, Zwei oder Drei mit der Maßgabe besitzen, daß $m + n \geqq 1$ ist,
- p Null oder eine ganze Zahl mit einem Wert von 1 bis 6, und
- -A-B- eine der Gruppen der Formel:

  $-(CH_2)_2-O-$; $-(CH_2)_3-O-$; $-CH=CH-$; $-CH_2-CH_2-$ und

$$-\underset{\underset{O}{\|}}{C}-CH=CH-$$

bedeuten,
in racemischer oder optisch aktiver Form,
und von deren physiologisch verträglichen Salzen mit geeigneten Säuren,
**dadurch gekennzeichnet**, daß man:

- ein N-monosubstituiertes Piperazin der allgemeinen Formel II:

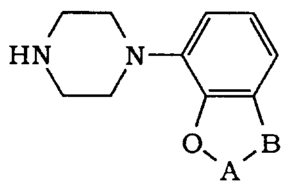

(II)

in der die Gruppe -A-B- die oben angegebenen Bedeutungen besitzt,
- mit einem Derivat der allgemeinen Formel III:

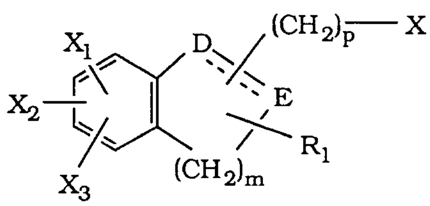

(III)

in der:
- $X_1$, $X_2$, $X_3$, $R_1$, -D⎓E- , m und p die oben angegebenen Bedeutungen besitzen und
- X ein Halogenatom oder eine Mesyloxy- oder Tosyloxygruppe darstellt, kondensiert;

- und gewünschtenfalls die in dieser Weise erhaltenen Derivate I mit geeigneten Säuren zur Bildung der entsprechenden Säureadditionssalze behandelt.

2. Verfahren nach Anspruch 1, **dadurch gekennzeichnet**, daß man die Kondensation der Derivate II und III in einem geeigneten Lösungsmittel bei einer Temperatur zwischen 20 und 150°C in Gegenwart eines Akzeptors für die im Verlaufe der Reaktion gebildete Säure durchführt.

3. Verfahren nach Anspruch 1 zur Herstellung der Derivate der allgemeinen Formel I':

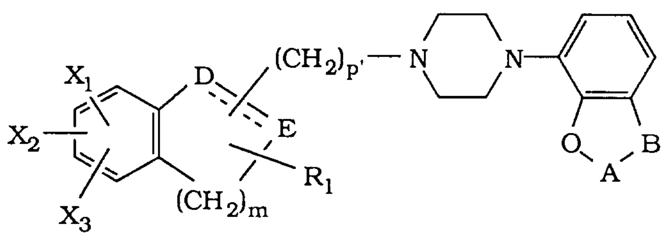

(I')

in der:
- $X_1$, $X_2$, $X_3$, $R_1$, -D⎓E- , m und -A-B- die in Anspruch 1 angegebenen Bedeutungen besitzen und
- p' eine ganze Zahl mit einem Wert von 1 bis 6 darstellt,

**dadurch gekennzeichnet**, daß man
- das N-monosubstituierte Piperazin der in Anspruch 1 definierten allgemeinen Formel II mit
- einem Derivat der allgemeinen Formel IV:

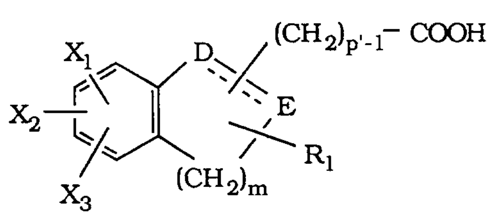

(IV)

in der:

- $X_1$, $X_2$, $X_3$. $R_1$, - D$\overline{\phantom{x}}$E- , m und p' die oben angegebenen Bedeutungen besitzen, kondensiert; und
- das in dieser Weise erhaltene Amid der allgemeinen Formel V:

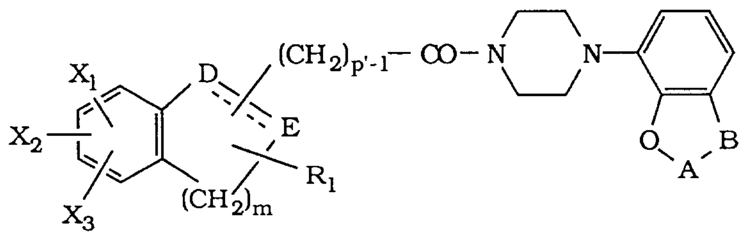

(V)

in der:

- $X_1$, $X_2$, $X_3$, $R_1$, -D$\overline{\phantom{x}}$E- , m, p' und -A-B- die oben angegebenen Bedeutungen besitzen, reduziert.

4. Verfahren nach Anspruch 3, **dadurch gekennzeichnet**, daß man die Kondensation der Derivate II und IV in Methylenchlorid und in Gegenwart von Carbonyldiimidazol bewirkt.

5. Verfahren nach Anspruch 3, **dadurch gekennzeichnet**, daß man die Reduktion des Amids V mit einem Lithium-Aluminium-Doppelhydrid in einem geeigneten Lösungsmittel bewirkt.

6. Verfahren zur Herstellung von Amiden der allgemeinen Formel V:

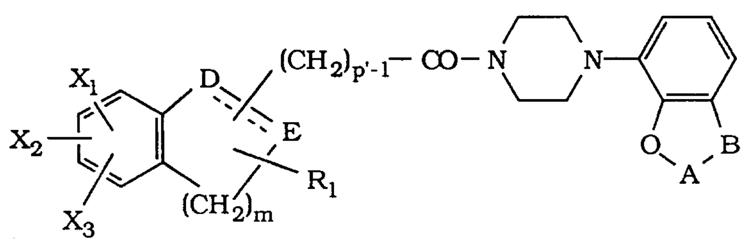

(V)

in der:

- $X_1$, $X_2$, $X_3$, $R_1$, -D$\overline{\phantom{x}}$E- , m, p' und -A-B- die in Anspruch 3 angegebenen Bedeutungen besitzen,

**dadurch gekennzeichnet**, daß man die Derivate II und IV, wie sie in Anspruch 3 definiert sind, kondensiert.